(19) Europäisches Patentamt
European Patent Office
Office européen des brevets

(11) Publication number: **0 000 816**
**A1**

(12) **EUROPEAN PATENT APPLICATION**

(21) Application number: 78300190.2

(22) Date of filing: 25.07.78

(51) Int. Cl.[2]: **C 07 D 213/74,** C 07 D 213/80
C 07 D 213/85 C 07 D 401/04
A 61 K 31/44 A 61 K 31/455
//(C07D401/04,257/04,213/74)

(30) Priority: 06.08.77 GB 33055/77
13.05.78 GB 19406/78

(43) date of publication of application:
21.02.79 Bulletin 79/4

(84) Designated contracting states:
BE CH DE FR GB NL SE

(71) Applicant: BEECHAM GROUP LIMITED
Beecham House Great West Road
Brentford, Middlesex(GB)

(72) Inventor: Baggaley, Keith Howard, Dr.
3 Blackstone Hill
Redhill Surrey(GB)

(72) Inventor: Thorne, David Edward, Dr.
"Russets" The Ridgeway
Cranleigh Surrey(GB)

(72) Inventor: White, Susan Mary
166 Station Avenue
West Ewell Epsom Surrey(GB)

(74) Representative: Hesketh, Alan, Dr. et al,
Beecham Pharmaceuticals Yew Tree Bottom Road
Epsom, Surrey, KT18 5XQ(GB)

(54) **Substituted amino-pyridine derivatives, processes for their preparation and pharmaceutical compositions containing them.**

(57) A class of substituted amino pyridine derivatives are of value in the treatment of diabetes. Some of the compounds also possess hypolipidaemic activity.

The compounds are represented by the formula (I):

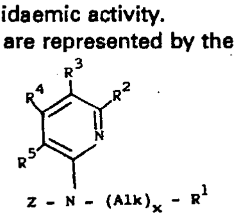

(I)

wherein $R^3$ is hydrogen, alkyl or an acidic function;
$R^2$ is hydrogen or alkyl;
$R^4$ and $R^5$ are hydrogen, alkyl or halogen;
Z is hydrogen, phenyl, alkyl or aralkyl;
Alk is alkylene and x is 0 or 1;
$R^1$ is optionally substituted phenyl or naphthyl.

Most of the compounds of formula (I) are novel.

EP 0 000 816 A1

TITLE MODIFIED
see front page



Biologically Active Compositions

This invention relates to pharmaceutical compositions which are of value in the treatment of diabetes. The active ingredients in the compositions comprise a class of substituted amino pyridine derivatives, many of which are novel compounds and also form part of the invention. The active ingredients have hypoglycaemic activity. Some also possess hypolipidaemic and/or antilipolytic activity.

A number of substituted amino pyridine derivatives are known but no biological activity has been described for them. For example, U.S. patent no. 3,624,096 discloses compounds of formula:

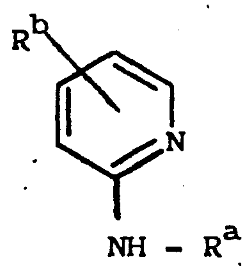

in which $R^a$ represents _inter alia_ an aryl group, and $R^b$ represents hydrogen, an alkyl group, a carboxy group, an ester group or a halogen atom. The only utility described for those compounds are either as chemical intermediates or as oxidation inhibitors.

In addition, 6-benzylamino-3-methylpyridine, and 6-benzylamino-4-methylpyridine are known from Chem. Tech. (Berlin)

10,693-9 (1958), but again no biological activity is disclosed therefor.

The present invention provides a pharmaceutical composition which comprises a pharmaceutically acceptable carrier together with at least one compound of formula (I):

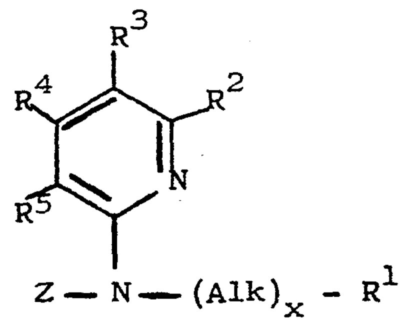

(I)

wherein $R^3$ is hydrogen or a carboxylic acid group or a pharmaceutically acceptable salt or ester of a carboxylic acid group; an alkyl group optionally substituted with one or more hydroxyl groups; or nitrile, formyl, tetrazolyl, or $C_{1-6}$ alkylcarbonyl group; and $R^2$ is hydrogen or $C_{1-6}$ alkyl and $R^4$ and $R^5$ are hydrogen, $C_{1-6}$ alkyl or halogen.

Z represents hydrogen, phenyl or $C_{1-6}$ alkyl optionally substituted with phenyl;

Alk represents a straight or branched chain alkylene group having up to 12 carbon atoms;

x is zero or 1; and

$R^1$ represents phenyl or naphthyl, optionally substituted with up to three groups selected from $C_{1-6}$ alkyl, phenyl, halogen, $C_{1-6}$ alkoxy, amino, nitro, hydroxy, $C_{1-6}$ alkylamido, $C_{1-6}$ alkylcarbonyloxy, carboxy, $C_{1-6}$ alkoxycarbonyl, halo($C_{1-6}$)alkyl, oxo ($C_{1-6}$)alkyl, or a pharmaceutically acceptable acid addition salt thereof.

Suitable ester groups for $R^3$ include groups of formula $CO_2R^o$, wherein $R^o$ is:

(a) $C_{1-20}$ alkyl, $C_{2-8}$ alkenyl or $C_{2-8}$ alkynyl each of which may be optionally substituted by $C_{3-7}$ cycloalkyl, halogen, carboxy, $C_{1-6}$ alkoxycarbonyl, carbamoyl, aryl, heterocyclyl,

hydroxy, $C_{1-6}$ alkanoyloxy, amino mono- and di- $(C_{1-6})$ alkylamino;

(b) $C_{3-7}$ cycloalkyl optionally substituted with $C_{1-6}$ alkyl;

(c) aryl;

(d) heterocyclyl;

(e) a group known to readily hydrolyse in the human body to produce the parent acid.

The term "aryl" includes phenyl and naphthyl optionally substituted with up to five halogen, $C_{1-6}$ alkyl, $C_{1-6}$ alkoxy, halo $(C_{1-6})$ alkyl, hydroxy, amino, carboxy, $C_{1-6}$ alkoxycarbonyl, or $C_{1-6}$ alkoxycarbonyl-$(C_{1-6})$-alkyl groups.

The term "heterocyclyl" includes single or fused rings comprising up to four hetero atoms in the ring selected from oxygen, nitrogen and sulphur and optionally substituted with up to three halogen, $C_{1-6}$ alkyl, $C_{1-6}$ alkoxy, halo-$(C_{1-6})$-alkyl, hydroxy, amino, carboxy, $C_{1-6}$ alkoxycarbonyl, $C_{1-6}$ alkoxycarbonyl $(C_{1-6})$ alkyl, aryl or oxo groups.

Thus the group $R^{o}$ may be for example $C_{1-6}$ alkyl, in particular, methyl, ethyl <u>n</u>- or <u>iso</u>-propyl, <u>n</u>-, <u>sec</u>-, <u>iso</u>- or <u>tert</u>-butyl; halo-$(C_{1-6})$-alkyl such as trifluoromethyl, 2-chloroethyl, 2,2,2-trichloroethyl; aminoalkyl groups such as aminoethyl, 2-aminoethyl; hydroxymethyl, 2-hydroxyethyl; phenyl; substituted phenyl; or a benzyl group

Examples of groups which hydrolyse readily in the body to produce the parent acid include acyloxyalkyl groups such as acetoxymethyl, pivaloyloxymethyl, α-acetoxyethyl, α-acetoxybenzyl and α-pivaloyloxyethyl groups; alkoxycarbonyloxyalkyl groups, such as ethoxycarbonyloxymethyl and α-ethoxycarbonyloxyethyl; dialkylaminoalkyl groups such as dimethylaminomethyl, dimethylaminoethyl, diethylaminomethyl or diethylaminoethyl; and lactone groups such as phthalidyl.

Suitable carboxylic acid salts include alkali metal salts such as lithium, sodium and potassium, other metal salts such as barium, calcium, aluminium or ammonium or substituted ammonium salts.

The alkyl group within the definition of $R^3$ may suitably have from 1 to 10 carbon atoms, such as methyl, ethyl, straight or branched chain propyl, butyl, pentyl, hexyl; and may be substituted at any position with one or more hydroxy groups.

Suitable alkylcarbonyl groups for $R^3$ include acetyl, propionyl and butyryl.

Suitably, the group $R^3$ is other than hydrogen. Advantageously, $R^3$ is carboxylic acid or a salt or ester thereof or alkyl.

Suitable alkyl groups for $R^2$, $R^4$ and $R^5$ and also for Z include methyl, ethyl and straight or branched chain propyl and butyl. Suitable halogen groups for $R^4$ and $R^5$ are chlorine, fluorine, bromine. Preferably, $R^4$ and $R^5$ are methyl, hydrogen or halogen. If halogen is present it is suitably at position $R^5$. Preferably Z is hydrogen or $C_{1-6}$ alkyl.

The group "Alk" may suitably be a $C_{1-10}$ alkylene chain, more suitably $C_{1-6}$ alkylene such as methylene, ethylene, propylene, butylene, optionally substituted by methyl or ethyl Preferably "Alk" represents straight chain alkylene such as methylene or ethylene.

Suitable substituents for the group $R^1$ include methyl, ethyl, n- and iso- propyl, n-, iso-, sec- and t- butyl, phenyl, chlorine, bromine, fluorine, iodine, methoxy, ethoxy, n- and iso- propoxy, n-, sec- and t- butoxy, carboxy, methoxy-carbonyl, ethoxycarbonyl, trifluoromethyl, 2,2,2-trichloroethyl, amino, nitro, hydroxy, acetamido ($-NHCOCH_3$), propionamido, acetoxy, formyl, formylmethyl, acetyl, acetylmethyl.

Suitable acid addition salts of compound (I) include inorganic salts such as the sulphate, nitrate, phosphate and borate, hydrohalides e.g. hydrochloride, hydrobromide and hydroiodide and organic acid addition salts such as acetate, oxalate, tartrate, maleate, citrate, succinate, benzoate, ascorbate, methanesulphate and p-toluenesulphonate.

One sub-class of compounds for use in the compositions of this invention has formula (II):

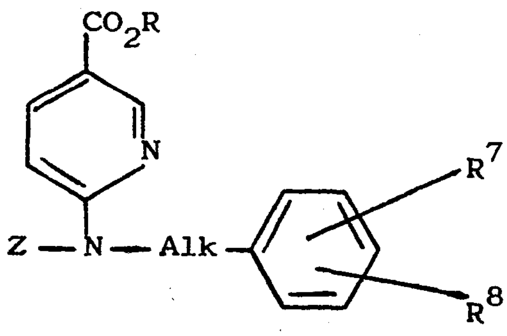

(II)

wherein Z and Alk are as defined above with respect to formula (I);

R represents hydrogen, a pharmaceutically acceptable salting ion, a $C_{1-6}$ alkyl group or a readily hydrolysable ester; and $R^7$ and $R^8$ represent hydrogen, halogen, $C_{1-6}$ alkyl, $C_{1-6}$ alkoxy, carboxy, $C_{1-6}$ alkoxycarbonyl, or nitro.

preferably $R^7$ is hydrogen, and $R^8$ is hydrogen, halogen, $C_{1-6}$ alkyl or $C_{1-6}$ alkoxy. Particular compounds within formula (II) in which Alk represents $CH_2$ include the following:

6-benzylaminonicotinic acid;
6-(N-benzyl-N-methylamino)-nicotinic acid;
6-(N-benzyl-N-ethylamino)-nicotinic acid;
6-(4-chlorobenzylamino)-nicotinic acid;
ethyl 6-(benzylamino) nicotinate;
6-(2-chlorobenzylamino)-nicotinic acid;
6-(4-nitrobenzylamino)-nicotinic acid;
6-(3-methylbenzylamino)-nicotinic acid;
6-(N,N-dibenzylamino)-nicotinic acid;
6-(3-chlorobenzylamino)-nicotinic acid;
6-(3,4-dichlorobenzylamino)-nicotinic acid;
6-(2-methylbenzylamino)-nicotinic acid;
6-(4-methylbenzylamino)-nicotinic acid;
6-(4-methoxybenzylamino)-nicotinic acid;
6-(3-methoxybenzylamino)-nicotinic acid;
6-(2-methoxybenzylamino)-nicotinic acid;
6-[N-methyl-N-(4-methylbenzyl)amino]-nicotinic acid;
ethyl 6-(4-methylbenzylamino)nicotinate;
6-[N-methyl-N-(3,4-dimethylbenzyl)amino]nicotinic acid;

BAD ORIGINAL

6-[N-methyl-N-(4-carboxybenzyl)amino]nicotinic acid;
6-(4-carboxybenzylamino)-nicotinic acid;
ethyl-6-[N-methyl-N-(4-ethoxycarbonyl)amino]-nicotinate;
ethyl-6-(4-ethoxycarbonylbenzylamino)-nicotinate;

methyl 6-[N-methyl-N-(4-methylbenzyl)amino]nicotinate
6-(3,4-dimethylbenzylamino)-nicotinic acid;
methyl 6-(4-methylbenzylamino)-nicotinate.

Compounds of formula (II) in which "Alk" represents an alkylene chain having from 2 to 6 carbon atoms, include the following:

1-(-)-6-(1-phenylethylamino)-nicotinic acid;
d-(+)-6-(1-phenylethylamino)-nicotinic acid;
6-(2-phenylethylamino)-nicotinic acid;
6-(3-phenylpropylamino)-nicotinic acid;
6-[N-methyl-N-(2-phenylethyl)amino]-nicotinic acid;
6-(4-phenyl-n-butylamino)-nicotinic acid;
6-[2-(4-methylphenyl)-ethylamino]-nicotinic acid
6-(5-phenyl-n-pentylamino)-nicotinic acid;
6-[N-methyl-N-(4-phenyl-n-butyl)amino]-nicotinic acid;
6-[N-methyl-N-(3-phenyl-n-propyl)amino]nicotinic acid;
6-[N-methyl-N-(4-methylphenethyl)amino]nicotinic acid;
6-[N-methyl-N-(5-phenyl-n-pentyl)amino]nicotinic acid;
6-(4-methoxyphenylethylamino)-nicotinic acid;
6-(4-bromophenylethylamino)-nicotinic acid;
6-[N-methyl-N-(4-bromophenethyl)amino]nicotinic acid;
6-[N-methyl-N-(4-methoxyphenylethyl)amino]nicotinic acid;
ethyl 6-(3-phenyl-n-propylamino)-nicotinate.

Another sub-class of compounds are those of formula (I) wherein $R^5$ is halogen. Particular such compounds include:

6-benzylamino-5-chloro-3-picoline
6-(4-methylbenzylamino)-5-chloro-3-picoline
6-(N-methyl-N-(4-methylbenzyl)amino)-5-chloro-3-picoline

Another group of compounds useful in the composition of this invention is represented by formula (III):

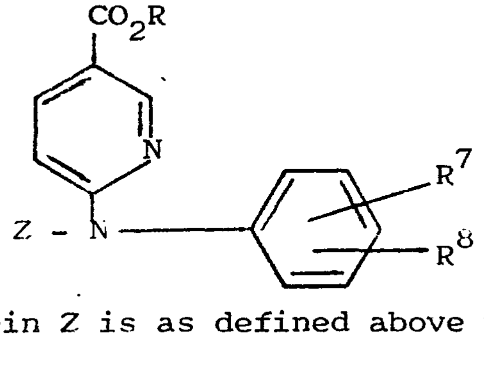

(III)

wherein Z is as defined above with respect to formula (I),

R represents hydrogen, a pharmaceutically acceptable salting ion, a $C_{1-6}$ alkyl group or a readily hydrolysable ester; and $R^7$ and $R^8$ represent hydrogen, halogen, $C_{1-6}$ alkyl, $C_{1-6}$ alkoxy, nitro, carboxy or $C_{1-6}$ alkoxycarbonyl. Preferably $R^7$ and $R^8$ represent hydrogen, halogen, $C_{1-6}$ alkyl or $C_{1-6}$ alkoxy.

Particular compounds of formula (III) include:

6-(phenylamino)-nicotinic acid;
6-(4-methylphenylamino)-nicotinic acid;
6-(N-ethyl-N-phenylamino)-nicotinic acid;
6-(N-methyl-N-phenylamino)-nicotinic acid;
6-(N,N-diphenylamino)-nicotinic acid;
6-(3-methylphenylamino)-nicotinic acid;
6-(4-chlorophenylamino)-nicotinic acid;
6-(4-ethylphenylamino)-nicotinic acid;
6-(4-bromophenylamino)-nicotinic acid;
6-(4-methoxyphenylamino)-nicotinic acid;
6-(3-chlorophenylamino)-nicotinic acid;
6-(4-fluorophenylamino)-nicotinic acid;
6-(4-iodophenylamino)-nicotinic acid;
6-(2,4-dichlorophenylamino)-nicotinic acid;
6-(2-chlorophenylamino)-nicotinic acid;
ethyl 6-(phenylamino)-nicotinate;
6-(3,4-dichlorophenylamino)-nicotinic acid;
6-(3,4-dimethylphenylamino)-nicotinic acid;
6-(3-methoxyphenylamino)-nicotinic acid;
6-(2-methylphenylamino)-nicotinic acid;
6-(2-methoxyphenylamino)-nicotinic acid;
6-(n-phenyl-N-_n_-propylamino)-nicotinic acid;
6-(4-ethoxycarbonylphenylamino)-nicotinic acid;
6-(4-nitrophenylamino)-nicotinic acid;
6-(4-carboxyphenylamino)-nicotinic acid;
6-(3,5-dichlorophenylamino)-nicotinic acid;
6-[N-methyl-N-(4-chlorophenyl)amino]nicotinic acid;
6-[N-methyl-N-(4-methylphenyl)amino]nicotinic acid.

A further sub-group of compounds within formula (I) above comprises compounds of formula (IV):

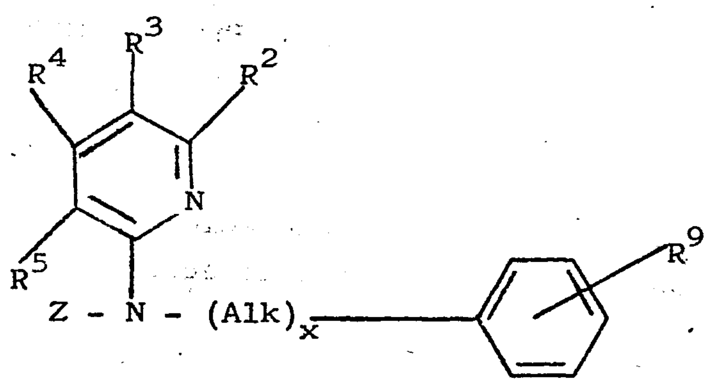

(IV)

wherein Z, Alk and x are as defined above with respect to formula (I), $R^2$, $R^3$ , $R^4$ and $R^5$ are hydrogen, or $C_{1-6}$ alkyl and $R^9$ is hydrogen, halogen, $C_{1-6}$ alkyl , $C_{1-6}$ alkoxy, carboxy or trifluoromethyl.

Preferably at least one of $R^2$, $R^3$, $R^4$ and $R^5$ is $C_{1-6}$ alkyl, especially methyl.

Suitably Z is alkyl, benzyl or phenyl. Particular compounds of formula (IV) include:

6-[N-methyl-N-(2-phenylethyl)amino]-3-picoline;

6-(N-methyl-N-benzylamino)-3-picoline;

6-benzylamino-3-picoline;

6-benzylamino-2-picoline;

6-benzylamino-5-picoline;

6-(4-methylbenzylamino)-3-picoline;

6-benzylamino-4-picoline;

6-phenylamino-3-picoline;

6-(4-chlorophenylamino)-3-picoline;

6-(4-chlorobenzylamino)-3-picoline;

6-(4-methylphenylamino)-3-picoline;

phenylamino -2-pyridine;

benzylamino-2-pyridine;

6-phenylamino-2-picoline;

6-[N-(4-methylbenzyl)-N-methylamino]-3-picoline;

6-(4-phenyl-*n*-butylamino)-3-picoline;

6-(2-phenylethylamino)-3-picoline;

6-(4-ethoxycarbonylbenzylamino)-3-picoline;

6-[N-methyl-N-(4-ethoxycarbonylbenzyl)amino]-3-picoline;

6-(4-carboxybenzylamino)-3-picoline;

6-[N-methyl-N-(4-carboxybenzyl)amino]-3-picoline;
6-(3-trifluoromethylbenzylamine)-3-picoline;
6-(4-bromobenzylamino)-3-picoline;

6-(4-methoxybenzylamino)-3-picoline;
6-[N-methyl-N-(4-methoxybenzyl)amino]-3-picoline;
6-(4-methylphenylethylamino)-3-picoline;
6-[N-methyl-N-(4-phenyl-n-butyl)amino]-3-picoline;
6-[N-methyl-N-(4-methylphenylethyl)amino]-3-picoline;
6-[N-methyl-N-(4-chlorobenzyl)amino]-3-picoline;
6-(5-phenyl-n-pentylamino)-3-picoline;
6-[N-methyl-N-(5-phenyl-n-pentyl)amino]-3-picoline;
6-(3-phenyl-n-propylamino)-3-picoline;
6-[N-methyl-N-(4-chlorophenyl)amino]-3-picoline;
6-[N-methyl-N-(3-phenyl-n-propyl)amino]-3-picoline;
6-[N-methyl-N-(3,4-dimethylbenzyl)amino]-3-picoline;
6-(3,4-dimethylbenzylamino)-3-picoline;
(N-ethyl-N-benzyl)amino-2-pyridine;
6-[N-ethyl-N-(4-chlorobenzyl)amino]-3-picoline;
6-[N-ethyl-N-(4-methylbenzyl)amino]-3-picoline;
6-[N-(n-propyl)-N-(4-methylbenzyl)amino]-3-picoline;
6-[N-(n-butyl)-N-(4-methylbenzyl)amino]-3-picoline;
6-[N-(iso-butyl)-N-(4-methylbenzyl)amino]-3-picoline.

Another sub-class of compounds of the present invention comprises compounds of formula (I) in which $R^3$ is a nitrile or tetrazole group. Examples of such compounds include:

6-benzylamino nicotinonitrile
5-[4-(benzylamino)-3-pyridyl]-tetrazole
3-hydroxymethyl-6-[N-methyl-N-(4-methylbenzyl)amino]-pyridine

The compounds for use in the compositions of this invention may be prepared by known methods, for example by the reaction of a compound (V) with a compound (VI):

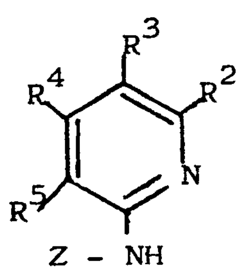

(V)

$$Hal - (Alk)_x - R^1$$

(VI)

wherein $R^1$, $R^2$, $R^3$, $R^4$, $R^5$, Z, Alk and x are as defined above and Hal represents halogen.

The compositions may be formulated for administration by any route, although an oral administration is preferred. The compositions may be in the form of tablets, capsules, powders, granules, lozenges, or liquid preparations, such as oral or sterile parenteral solutions or suspensions.

Tablets and capsules for oral administration may be in unit dose presentation form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine; tabletting lubricants, for example magnesium stearate, talc, polyethylene glycol, or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oilv suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, glucose syrup gelatin, hydroxyethylcelluslose, carboxy-methyl cellulose, aluminium stearate gel or hydrogenated edible fats, emulsifying agents, for example lecithin, sorbitan nonooleate, or acacia; non-aqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents. The compound may also if desired be incorporated in a foodstuff, for example in the form of a biscuit.

For parenteral administration, fluid unit dosage forms are prepared utilizing the compound and a sterile vehicle, water being preferred. The compound, depending on the vehicle

and concentration used, can be either suspended or dissolved in the vehicle. In preparing solutions the compound can be dissolved in water for injection and filter sterilized before filling into a suitable vial or ampoule and sealing. Advantageously, adjuvants such as a local anesthetic, preservative and buffering agents can be dissolved in the vehicle. To enhance the stability, the composition can be frozen after filling into the vial and the water removed under vacuum. Parenteral suspensions are prepared in substantially the same manner except that the compound is suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

The compositions may contain from 0.1% to 99% by weight, preferably from 10-60% by weight, of the active material, depending on the method of administration. The dosage employed for adult treatment will of course depend on the dose-response characteristics of the particular active ingredient but will normally be in the range 0.5 to 300 mg/kg/day.

Many of the compounds of formula (I) are novel compounds and, in a further aspect, this invention provides a compound of formula (VII):

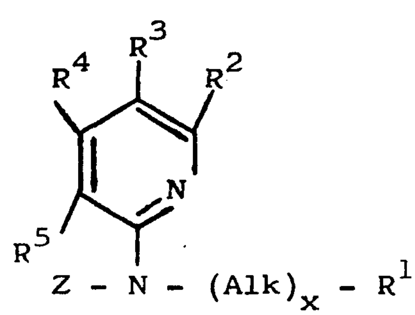

(VII)

wherein $R^1$, $R^2$, $R^3$, $R^4$, $R^5$, Alk, x, and Z are defined above with respect to formula (I), provided that:

- 12 -

(a) When x represents zero, then Z represents $C_{1-6}$ alkyl or phenyl; and

(b) When x represents 1, Z is hydrogen, Alk is $-CH_2-$ and either $R^3$ or $R^4$ is methyl, then $R^1$ is other than phenyl.

One group of novel compounds is represented by formula (II) above.

The novel compounds of this invention may be prepared by a process which comprises reacting an amine of formula (VIII) with a halide of formula (IX):

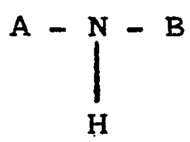

(VIII)

$$Hal - D$$

(IX)

wherein "Hal" represents halogen; one group A,B or D represents a group of formula (X):

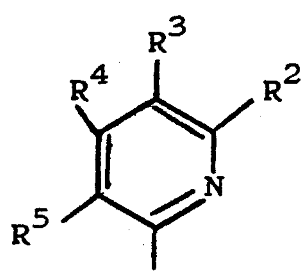

(X)

[wherein $R^2$, $R^3$, $R^4$ and $R^5$ are as defined with respect to formula (VII)]; one group A, B or D represents a group of formula (XI):

$$-(Alk)_x - R^1 \qquad (XI)$$

[wherein Alk, x and $R^1$ are as defined with respect to formula (VII)] and the third group A, B or D represents the

group Z as defined with respect to formula (VII) and optionally converting one group $R^1$ or $R^3$ to a different such group.

This reaction may be carried out in a solvent, for example a high boiling, inert organic solvent such as diethylene glycol, xylene, toluene, dimethylformamide, dimethylsulphoxide, dioxan, or water. Alternatively, the two reagents (VIII) and (IX) may be heated together in the absence of solvent. In either case, a high temperature is usually required, preferably at least 100°C. In some cases, especially when the group D is of formula (XI), more vigorous conditions are required and the reaction can be carried out in a sealed tube and/or in the presence of a catalyst such as copper, potassium iodide, sodium iodide, potassium carbonate, or sodamide.

Alternative methods of preparing compounds (VII) wherein $R^3$ is an ester group include the esterification of the free acid or its salt or other reactive derivative of the acid, or transesterification of a compound having a different ester group. Esterification may be performed by any conventional method, for example by reaction of the free acid with the appropriate alcohol in the presence of a catalyst such as a strong acid, dry hydrogen chloride, or p-toluenesulphonic acid.

The formation of compounds (VII) wherein $R^3$ is an ester may also be carried out by conventional transesterification methods, for example reaction of an ester with the appropriate second alcohol in the presence of a catalyst such as the sodium salt of the alcohol, or dry hydrogen chloride, p-toluenesulphonic acid, or potassium cyanide.

Compounds of formula (VII) wherein $R^3$ is an ester may also be prepared by alkanolysis of the corresponding cyano compound ($R^3$ is C≡N); or by hydrolysis of an iminoether compound having formula (VII) wherein $R^3$ is a group of formula:

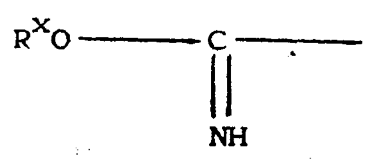

wherein $R^x$ is the hydrocarbon residue of an alcohol or phenol.

Compounds wherein $R^3$ is a carboxylic acid group can also be prepared by the acid or base catalysed hydrolysis of the corresponding compound of formula (I) wherein $R^3$ is selected from:

(a) carboxylic acid amide group:
(b) cyano group (-C≡N);
(c) esterified carboxylic acid group.

Hydrolysis of amides may be carried out using a mineral acid as catalyst, suitably hydrochloric acid or sulphuric acid. Base catalysed hydrolysis may be carried out using an alkali metal or alkaline earth metal hydroxide, e.g. sodium or potassium hydroxide. Suitably the hydrolysis reaction is carried out in aqueous solution e.g. refluxing for several hours. The desired compound can be isolated as the free acid by neutralisation of the resultant reaction mixture or as the appropriate base addition salt (e.g. sodium salt if sodium hydroxide was employed) or acid addition salt (e.g. the hydrochloride if HCl was employed.) Alternatively the free acid can be converted to any desired salt by standard procedures.

For the hydrolysis of a compound wherein $R^3$ is a cyano group, ammonia is liberated and thus the preferred catalyst is an acid which will bind the ammonia e.g. hydrogen halide such as HCl or HBr. If base catalysed hydrolysis is used, ammonia is liberated and the acid will be obtained as an alkali salt, or after neutralisation, as the free acid.

For the hydrolysis of an esterified carboxylic acid group, preferably the process involves hydrolysis with a strong base such as sodium hydroxide. The esterified carboxylic acid groups $R^3$ may be, for example lower alkoxycarbonyl groups such as methoxycarbonyl or tertiary butoxycarbonyl groups. The remarks made earlier about salts of the resultant free acid also apply in this case.

Compounds wherein $R^3$ is a tetrazolyl group may be prepared from a compound in which $R^3$ is a cyano group, for example by treatment with sodium azide, ammonium chloride and lithium bromide.

Compounds of formula (VII) may also be prepared by reacting an amine of formula (XII) with a compound of formula (XIII):

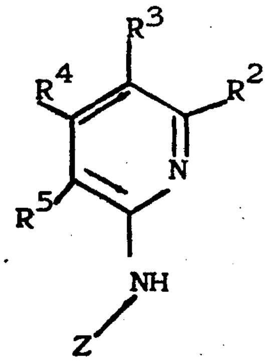

(XII)

$$HO - (Alk)_x - R^1$$

(XIII)

This reaction is generally carried out in the presence of a base, e.g. sodium or potassium hydroxide, preferably without additional solvent at elevated temperature.

Compounds of formula (VII) wherein x is 1 and Alk represents methylene may be prepared by reducing a compound of formula (XIV):

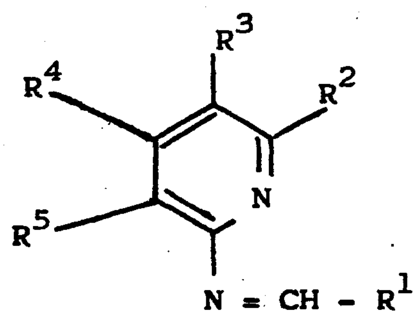

(XIV)

Suitable reagents for this reduction include metallic hydrides, in particular sodium borohydride in an alcohol such as ethanol.

Compounds of formula (XIV) may be prepared by condensing together compounds of formula (XV) and (XVI):

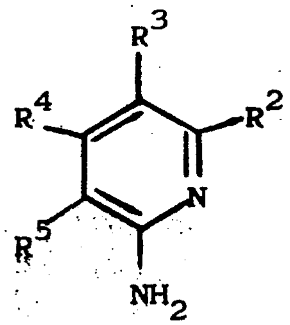

$R^1 - CHO$

(XV) (XVI)

Compounds of formula (VII) wherein Z is methyl may be prepared by reacting a compound of formula (VII) wherein Z is hydrogen with a mixture of formic acid and formaldehyde.

Compounds of formula (VII) with a $CH_2$ group adjacent the amino nitrogen atom may be prepared by reducing a ketone of formula (XVIIA) or (XVIIB):

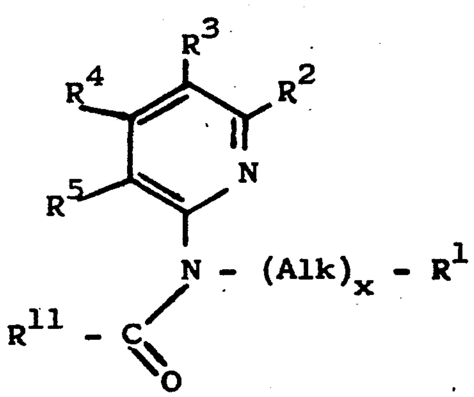

(XVIIA)

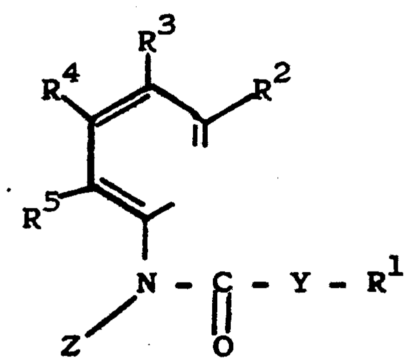

(XVIIB)

wherein $R^{11}$ represents a $C_1$-$C_5$ alkyl group optionally substituted with phenyl and Y represents a bond or a $C_1$-$C_{11}$ alkylene group. Suitable reagents for this reduction are those capable of reducing amides, for example, lithium aluminium hydride, diborane or preferably the reagent of formula $BH_3.S(CH_3)_2$.

It may be preferable to modify the substituents $R^1$ and/or $R^3$ after the condensation reaction rather than before. Thus, it is preferable, when preparing compounds of formula (VII) wherein $R^3$ is a carboxylic acid group, first to prepare the corresponding compound with a carboxylic acid ester group and then to convert such group to carboxylic acid group by conventional means.

Compounds of formula (VII) wherein $R^3$ is a hydroxymethyl group may be prepared by reduction of a compound (VII) wherein $R^3$ is a formyl or carboxylic ester group. One reagent suitable for this reduction is lithium aluminium hydride.

The following examples illustrate the preparation of some of the novel compounds of this invention:

EXAMPLE 1

6-Benzylaminonicotinic Acid

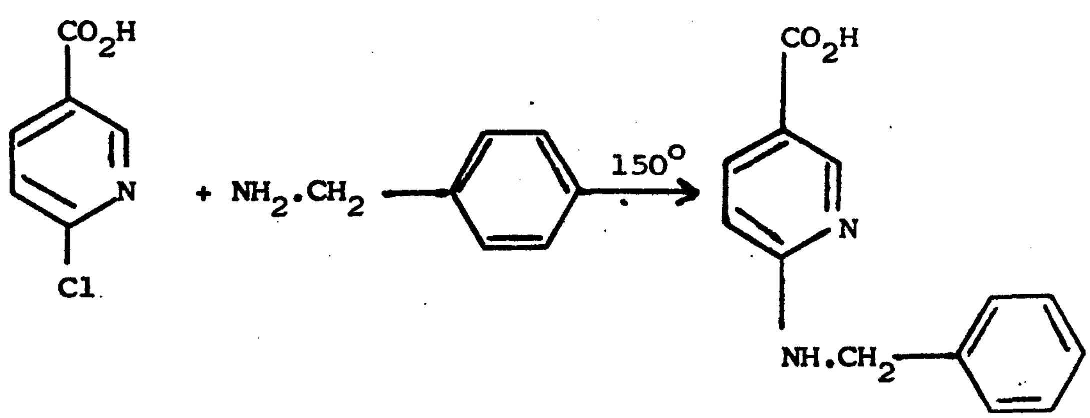

Two stoichiometric equivalents of benzylamine (6.8 g) were added to 6-chloro-nicotinic acid (5 g). The mixture was heated at 150°, with stirring, for 4 hours. The cooled reaction mixture was diluted with water. The resulting solid-liquid mixture was filtered. The solid was taken up in sodium hydroxide solution, heated, with stirring, for 15 minutes and filtered. The basic filtrate was brought to pH 6 by addition of dilute hydrochloric acid. A white precipitate was deposited. The precipitate was filtered off, washed with water, and dried, m.p. 228-230°C.

EXAMPLES 2-32

The following compounds were prepared by a method substantially as described in Example 1:

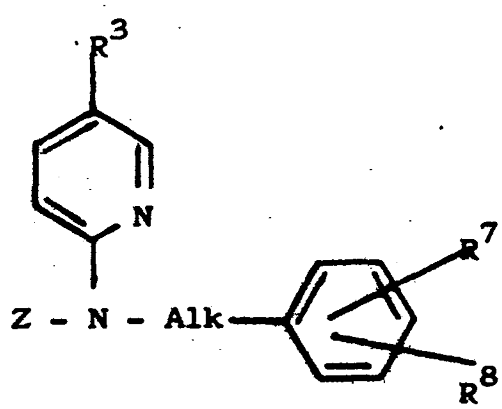

| Example No. | $R^3$ | Z | Alk | $R^7$, $R^8$ | m.p.(°C.) |
|---|---|---|---|---|---|
| 2 | $CO_2H$ | H | 1-(-)-$\overset{CH_3}{CH}$- | H | 233-235 |
| 3 | $CO_2H$ | $C_2H_5$ | - | H | 158-160 |
| 4 | $CO_2H$ | H | $(CH_2)_2$ | H | 215-218 |
| 5 | $CO_2H$ | $CH_3$ | - | H | 174-175 |
| 6 | $CO_2H$ | phenyl | - | H | 235-237 |
| 7 | $CO_2H$ | $CH_3$ | $CH_2$ | H | 192-193 |
| 8 | $CO_2H$ | $C_2H_5$ | $CH_2$ | H | 138-141 |
| 9 | $CO_2H$ | H | d-(+)-$\overset{CH_3}{CH}$ | H | 224-227 |
| 10 | $CO_2H$ | H | $CH_2$ | 4-Cl | 205-208 |
| 11 | $CO_2H$ | H | $(CH_2)_3$ | H | 199-200 |
| 12 | $CO_2H$ | H | $CH_2$ | 2-Cl | 205-208 |
| 13 | $CO_2H$ | $CH_3$ | $(CH_2)_2$ | H | 161-163 |
| 14 | $CO_2H$ | H | $CH_2$ | 4-$NO_2$ | 262-265 |
| 15 | $CO_2H$ | n-$C_3H_7$ | - | H | 135-138 |
| 16 | $CO_2H$ | H | $(CH_2)_4$ | H | 176-177 |
| 17 | $CO_2H$ | H | $CH_2$ | 3-$CH_3$ | 184-186 |
| 18 | $CO_2H$ | $C_6H_5.CH_2$ | $CH_2$ | H | 159-162 |
| 19 | CN | H | $CH_2$ | H | 127-129 |
| 20 | $CO_2H$ | H | $CH_2$ | 3-Cl | 194-196 |
| 21 | $CO_2H$ | H | $(CH_2)_2$ | 4-$CH_3$ | 234-236 |
| 22 | $CO_2H$ | H | $CH_2$ | 3,4-di-Cl | 214-216 |
| 23 | $CO_2H$ | H | $CH_2$ | 2-$CH_3$ | 228-230 |
| 24 | $CO_2H$ | H | $(CH_2)_5$ | H | 139-140 |
| 25 | $CO_2H$ | H | $CH_2$ | 4-$CH_3$ | 226-227 |
| 26 | $CO_2H$ | H | $CH_2$ | 4-$OCH_3$ | 223-226 |
| 27 | $CO_2H$ | H | $CH_2$ | 3-$OCH_3$ | 202-204 |
| 28 | $CO_2H$ | H | $CH_2$ | 2-$OCH_3$ | 188-190 |
| 29 | $CO_2C_2H_5$ | H | $CH_2$ | 4-$CH_3$ | 112-114 |
| 30 | $CO_2H$ | H | $(CH_2)_2$ | 4-$OCH_3$ | 201 |
| 31 | $CO_2H$ | H | $(CH_2)_2$ | 4-Br | 223-225 |
| 32 | $CO_2H$ | H | $CH_2$ | 3,4-di-$CH_3$ | 228-229 |

EXAMPLE 33

Preparation of 6-benzylamino-2-picoline

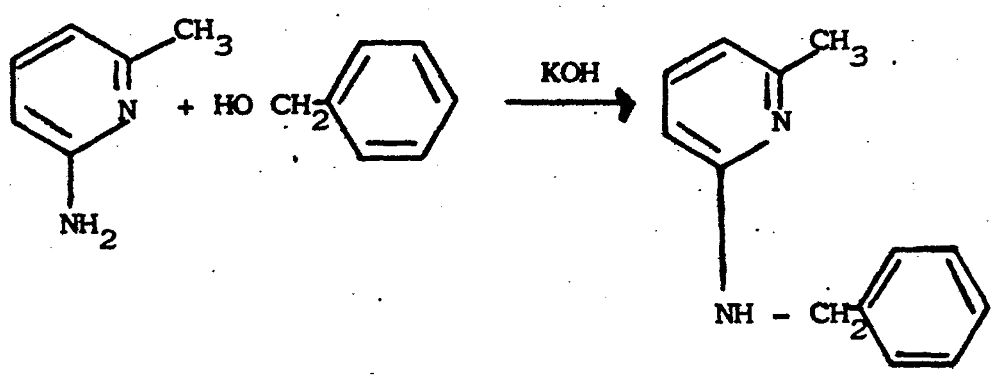


Method:

2-amino-6-picoline (10.8 g, 0.01M), benzyl alcohol (15 g) and 85% KOH (0.9 g) were placed in a 100 ml flask set up for distillation. A thermometer was placed such that its bulb was in the reaction mixture. The flask was heated on an electric mantle such that water distilled over slowly accompanied by as little benzyl alcohol as possible. The temperature of the boiling mixture rose gradually from 180° to 250° over a period of about 30 minutes. The mixture was maintained at 250° for 3 minutes and then allowed to cool. The contents of the flask were cooled to 100° and then poured into water (50 ml). The solid which immediately formed was crushed and collected on a Buchner funnel. The dried product was recrystallised from an ethanol/Pet. Ether (40-60) mixture. The product was a white crystalline material, m.p. 58-60°C.

EXAMPLES 34-44

The following compounds were prepared by a method substantially as described in Example 33.

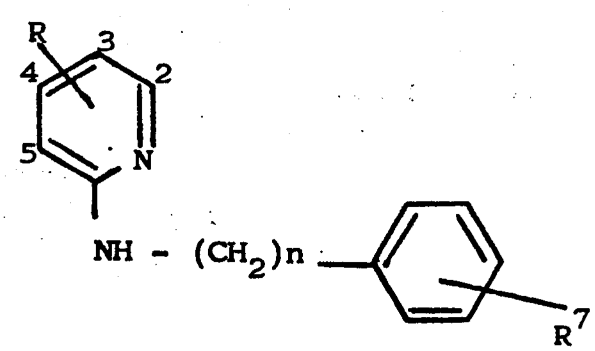

| Example No. | R | n | $R^7$ | m.p.(°C) | b.p. (mm.Hg) |
|---|---|---|---|---|---|
| 34 | H | 1 | H | 95-96 | |
| 35 | 5-$CH_3$ | 1 | H | | 130° (0.3) |
| 36 | 3-$CH_3$ | 1 | 4-$CH_3$ | 128-130 | |
| 37 | 3-$CH_3$ | 1 | 4-Cl | 159-162 | |
| 38 | 3-$CH_3$ | 1 | 4-$OCH_3$ | 143-144 | |
| 39 | 3-$CH_3$ | 5 | H | 54-55 | |
| 40 | 3-$CH_3$ | 3 | H | 55-56 | |
| 41 | 3-$CH_3$, 5-Cl | 1 | 4-$CH_3$ | | 210° (2.0) |
| 42 | 3-$CH_3$ | 1 | 3,4-di-$CH_3$ | 76-78 | |
| 43 | 3-$CH_3$ | 1 | 3-$CF_3$ | 78-79 | |
| 44 | 3-$CH_3$ | 1 | 4-Br | 160-162 | |

EXAMPLE 45

6-(2-Naphthylmethylamino)-3-picoline was prepared by the method of Example 33, m.p. 161-3°.

EXAMPLE 46

Preparation of 6-(4-ethoxycarbonylbenzyl)-amino-3-picoline

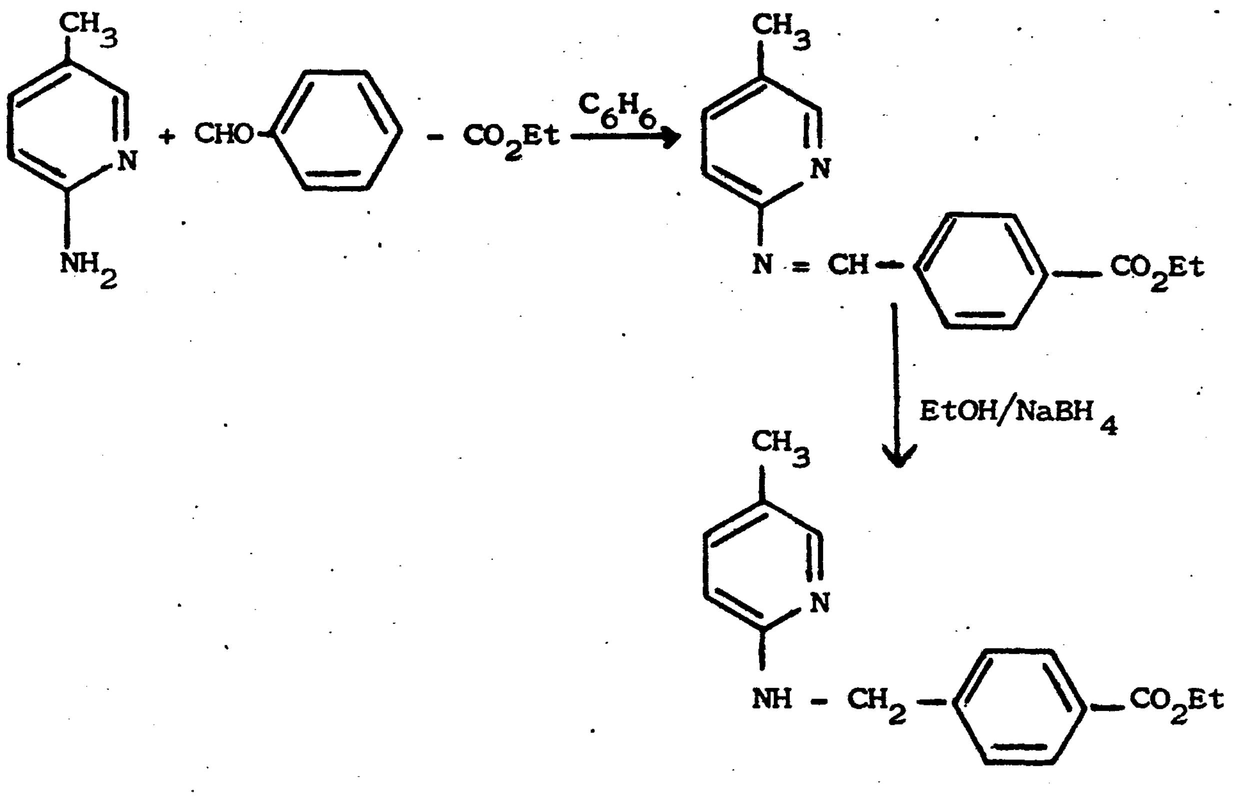


2-amino-5-picoline (5.4 g) was dissolved in dry benzene (100 ml). A slight excess of ethyl-4-carboxybenzaldehyde (9 g) was added and the mixture was heated under reflux for 30 hours using a Dean and Stark separator. The solvent was removed by rotary evaporation and the solid residue was taken up in ethanol (100 ml). Sodium borohydride (3.78 g) was added with caution, a vigorous reaction taking place. The resulting solution was refluxed for 2 hours. The solvent was removed and the residue dissolved in chloroform and washed with water. The chloroform phase was dried over $MgSO_4$ and evaporated to dryness. A pale yellow solid was obtained which was purified by column chromatography. (Silice Gel/Ether.) The product was isolated as a white crystalline solid; mass of product 6 g; m.p. 123-125°C.

EXAMPLES 47-48

The following compounds were prepared by a method substantially as described in Example 46.

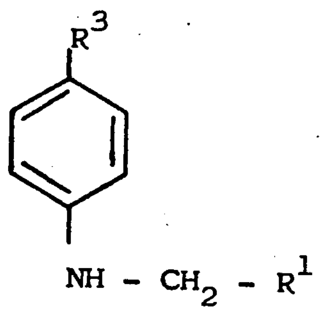

| Example No. | $R^3$ | $R^1$ | m.p. ($^oC$) |
|---|---|---|---|
| 47 | $CO_2Et$ | 4-ethoxycarbonylphenyl | 127-30 |
| 48 | $CH_3$ | 1-naphthyl | 107-108.5 |

EXAMPLE 49

Preparation of 6-(4-methylphenylethyl)amino-3-picoline

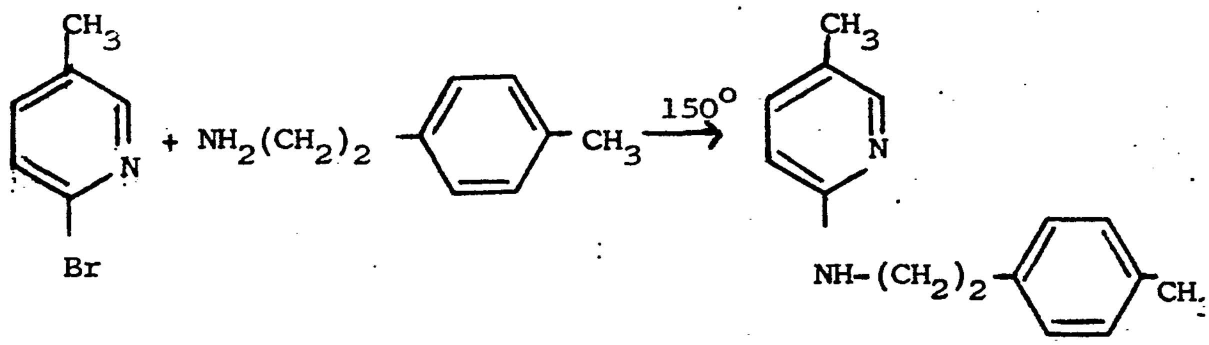


2-bromo-5-picoline (6.36 g) and p-tolyl ethylamine (10 g) were heated together at 150°C, with stirring, for 6 hours. The cooled reaction mixture was diluted with water, made basic with sodium hydroxide solution and extracted with ether. The ether extract was dried and concentrated to give a yellow oil. The oil was purified by distillation.

Boiling Range: 143-145°C (0.3 mm. Hg.)

EXAMPLES 50-51

The following compounds were prepared by a method substantially as described in Example 49.

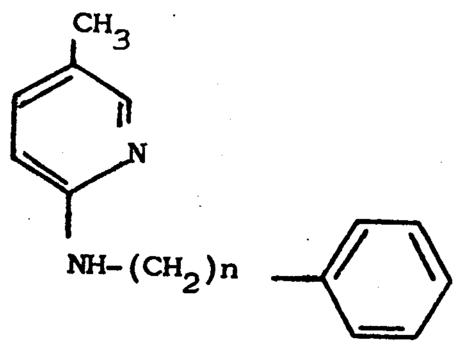

| Example No. | n | b.p. ($^{o}C$, m.m. Hg) |
|---|---|---|
| 50 | 4 | 166° (0.5mm) |
| 51 | 2 | 140-42° (0.3mm) |

EXAMPLE 52

Ethyl 6-benzylaminonicotinate

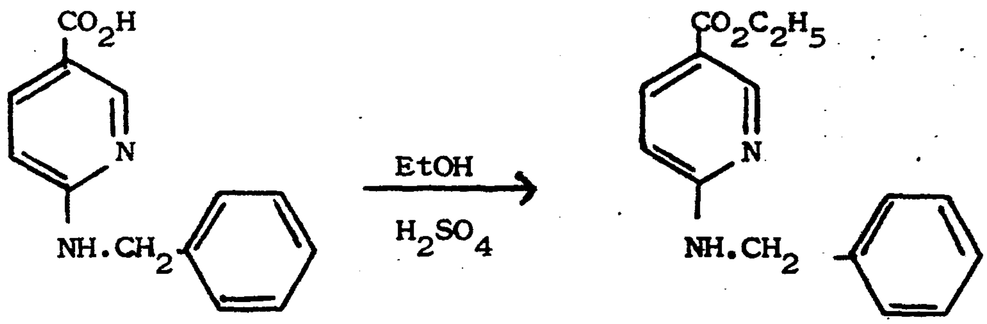


6-benzylaminonicotinic acid (4 g) was added to ethanol (100 ml) acidified with sulphuric acid. The mixture was refluxed for 6 hours. The cooled reaction mixture was poured into water and extracted with chloroform. The chloroform extract was dried over magnesium sulphate and evaporated to dryness. A pale-yellow, semi-solid residue remained. Infrared spectroscopy and thin layer chromatography showed that the residue was a mixture of the starting acid and the required ester. The mixture was passed through a chromatography column (alumina/$CHCl_3$). The ester was eluted first and was isolated as a white solid. The product was recrystallised from Ethanol/ Pet. ether (60-80), m.p. 95-97°.

EXAMPLES 53-54

The following compounds were prepared by a method substantially as described in Example 52.

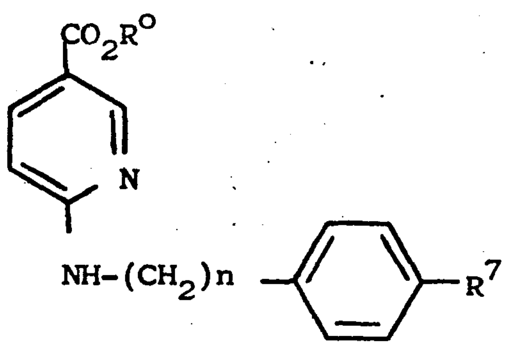

| Example No. | $R^o$ | n | $R^7$ | m.p. ($^oC$) |
|---|---|---|---|---|
| 53 | $C_2H_5$ | 3 | H | 67 |
| 54 | $CH_3$ | 1 | $CH_3$ | 133-134 |

EXAMPLE 55

Preparation of 6-(4-carboxybenzyl)amino-nicotinic acid

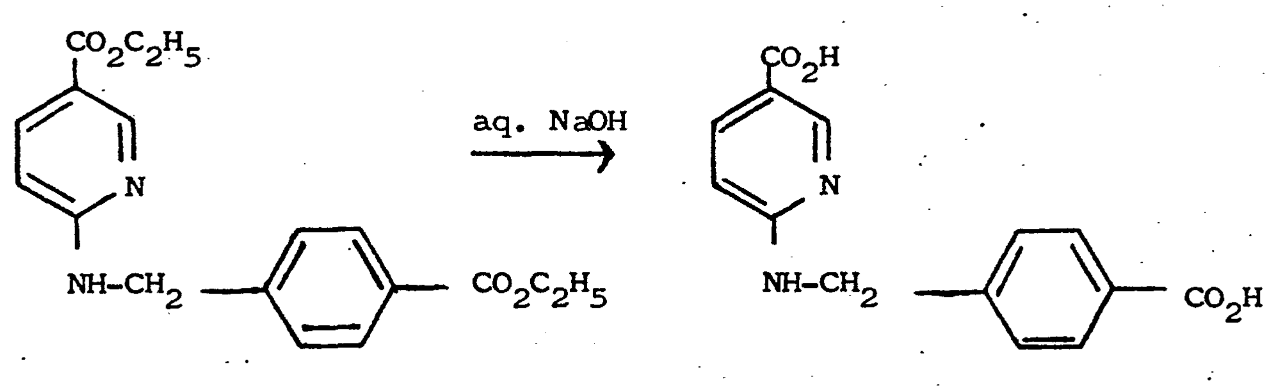


The di-ester (1.5 g) was heated under reflux in sodium hydroxide solution (50 ml, 1N soln.) for 6 hours. The reaction mixture was cooled and brought to pH5 with dilute HCl. A white precipitate was formed and filtered off. The solid was recrystallised from ethanol.

Melting Point: 297-300°C (dihydrate)

EXAMPLES 56-60

The following compounds were prepared by a method substantially as described in Example 55.

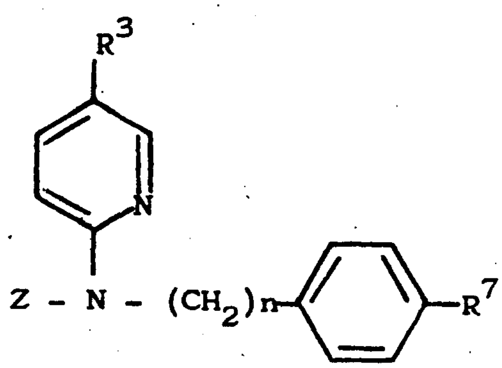

| Example No. | n | $R^3$ | Z | $R^7$ | m.p. (°C) |
|---|---|---|---|---|---|
| 56 | 1 | $CH_3$ | H | $CO_2H$ | 240-243 |
| 57 | 1 | $CH_3$ | $CH_3$ | $CO_2H$ | 144-147 |
| 58 | 1 | $CO_2H$ | H | $CO_2H$ | 275-278 |
| 59 | 1 | $CO_2H$ | $CH_3$ | $CO_2H$ | - |
| 60 | 0 | $CO_2H$ | $CH_3$ | Cl | 184-187 |

EXAMPLE 61

6-[N-methyl-N-(4-methylbenzyl)]-amino-3-picoline

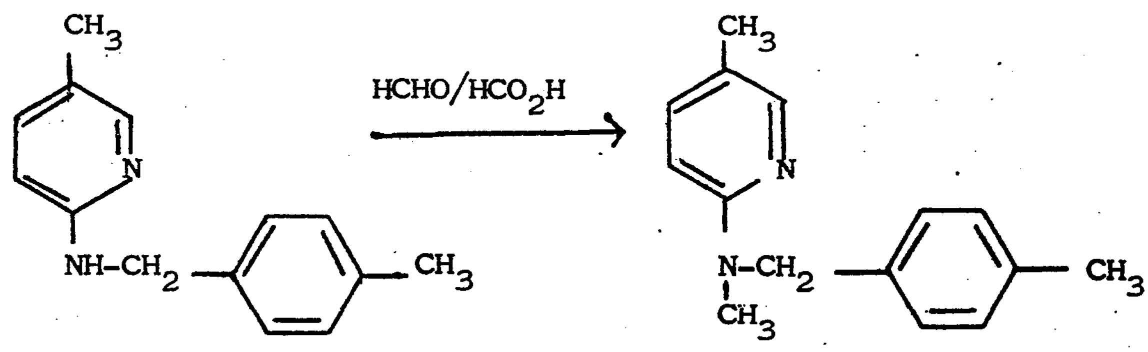


6-(4-methylbenzyl)-amino-3-picoline (5.25 g, 0.025M) was added to a mixture of formic acid (6.25 ml) and formaldehyde solution (3 ml 40% HCHO). The mixture was heated at 100° for eight hours. The cooled reaction mixture was poured into dilute sodium sulphite solution and made basic with NaOH solution. The product was extracted into ether and isolated as a yellow oil which was distilled under vacuum.

Mass of Product: 3.5 g

Yield: 63%

Boiling Point: 160° (0.3 mm Hg)

EXAMPLES 62-83

The following compounds were prepared by a method substantially as described in Example 61.

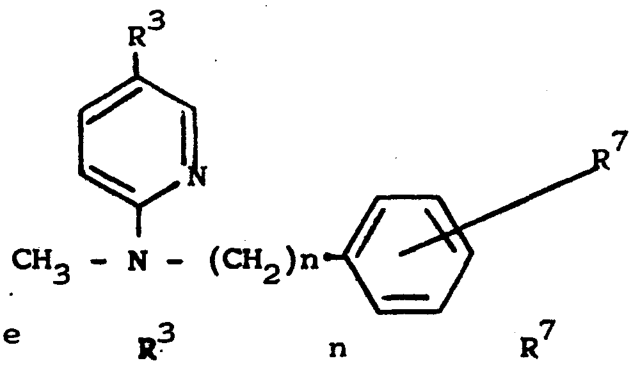

| Example No. | $R^3$ | n | $R^7$ | m.p. ($^oC$) | b.p. ($^oC$, mm Hg) |
|---|---|---|---|---|---|
| 62 | $CH_3$ | 1 | H | | $140^o$ (0.2 mm) |
| 63 | $CO_2H$ | 4 | H | 142-144 | |
| 64 | $CH_3$ | 2 | H | | $124^o$ (0.25 mm) |
| 65 | $CO_2H$ | 3 | H | 178-181 | |
| 66 | $CO_2H$ | 2 | 4-$CH_3$ | 139-141 | |
| 67 | $CO_2H$ | 5 | H | 145-147 | |
| 68 | $CO_2H$ | 1 | 4-$CH_3$ | 202-203 | |
| 69 | $CH_3$ | 1 | 4-$OCH_3$ | | $165^o$ (0.4 mm) |
| 70 | $CH_3$ | 4 | H | | $160^o$ (0.3 mm) |
| 71 | $CH_3$ | 2 | 4-$CH_3$ | | 140-$5^o$ (0.5 mm) |
| 72 | $CH_3$ | 1 | 4-Cl | | $145^o$ (0.5 mm) |
| 73 | $CO_2CH_3$ | 1 | 4-$CH_3$ | 55 | |
| 74 | $CH_3$ | 5 | H | | 195-$7^o$ (0.3 mm) |
| 75 | $CH_3$ | 0 | 4-Cl | | 150-$1^o$ (0.6 mm) |
| 76 | $CH_3$ | 3 | H | | 152-$5^o$ (0.6 mm) |
| 77 | $CO_2H$ | 2 | 4-$OCH_3$ | 116-120 | |
| 78 | $CH_3$ | 1 | 3,4-di-$CH_3$ | | |
| 79 | $CH_3$ | 1 | 4-$CO_2Et$ | | |
| 80 | $CO_2Et$ | 1 | 4-$CO_2Et$ | | |
| 81 | $CO_2H$ | 2 | 4-Br. | 167-169 | |
| 82 | $CO_2H$ | 0 | 4-$CH_3$ | 154-56 | |
| 83 | $CO_2H$ | 1 | 3,4-di-$CH_3$ | 187-189 | |

EXAMPLES 84-85

The following compounds were also prepared by a method substantially as described in Example 61.

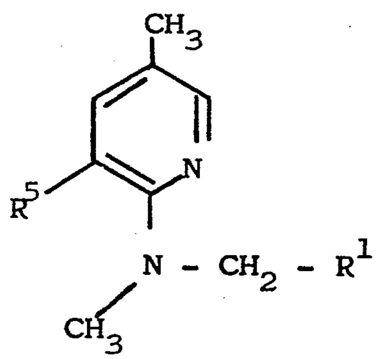

| Example No. | $R^5$ | $R^1$ | m.p. (°C) |
|---|---|---|---|
| 84 | Cl | 4-methylphenyl | |
| 85 | H | 1-naphthyl | 53.5-54.5 |

EXAMPLE 86

5-[4-(benzylamino)-3-pyridyl]-tetrazole

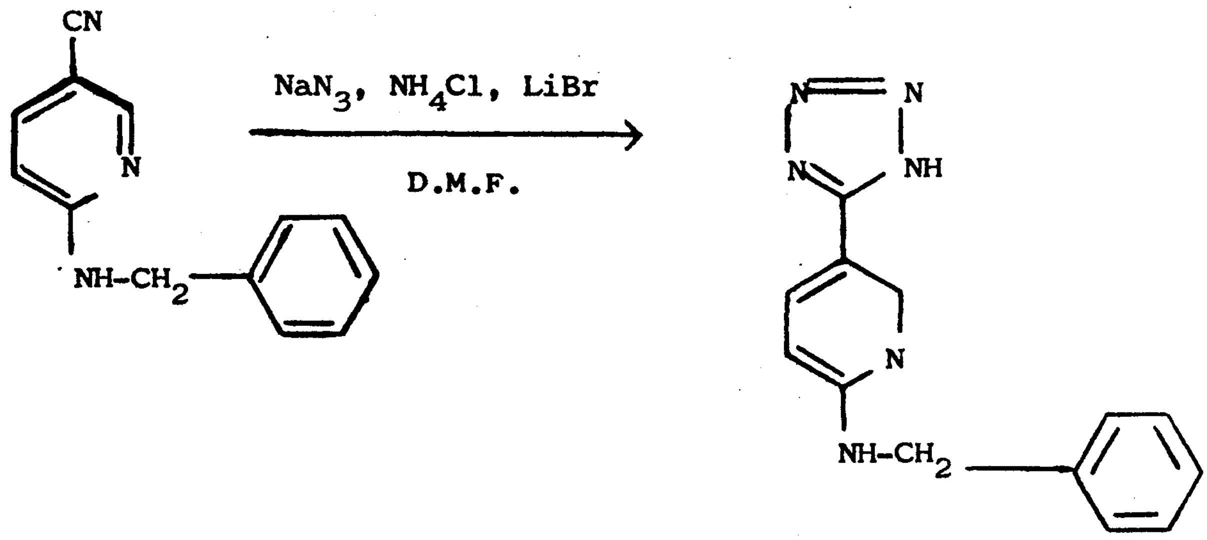


6-Benzylamino nicotinonitrile (2.34 g, 0.011 M) was added to a mixture of sodium azide (0.975 g), ammonium chloride (0.81 g) and lithium bromide (0.015 g) in dimethylformamide (7.5 ml). The mixture was heated at 125°C for twelve hours. When cool, the insoluble material was removed by filtration and the filtrate was concentrated under reduced pressure to give an orange gum. A yellow solid formed on addition of water. The solid was reprecipitated from basic solution with HCl. The product was recrystallised from ethanol as the hydrate.

Mass of Product: 1.3 g

Yield: 45%

Melting Point: 204-205%

EXAMPLE 87

2-[N-benzyl-N-ethyl]amino pyridine

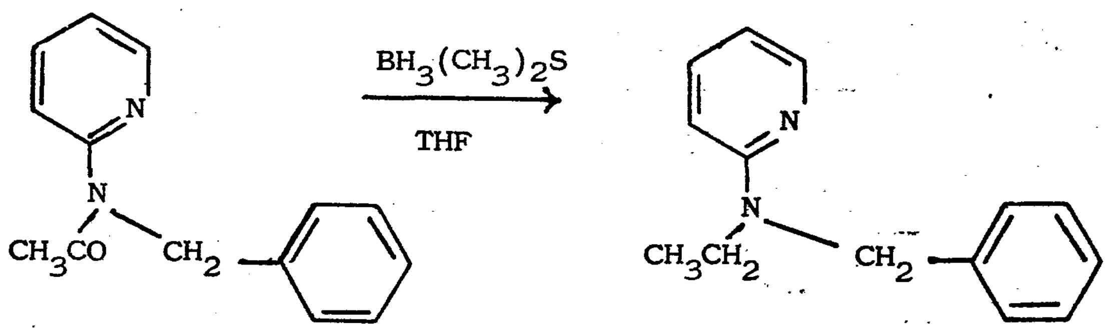


Borane - methyl sulphide complex (3.8 ml) in dry tetrahydrafuran (25 ml) was added dropwise, at room temperature, to 2-[N-acetyl-N-benzyl]amino pyridine (4.7 g) in dry tetrahydrofuran under nitrogen with stirring over 40 minutes. After a further 20 minutes, the mixture was reflux/stirred under nitrogen for 5 hours and cooled to room temperature. Methanol (7 ml) in dry tetrahydrofuran (10 ml) was added over one hour, allowed to stand overnight, cooled to 0°C, hydrogen chloride passed through for 25 minutes then heated under reflux for one hour, cooled and evaporated to dryness. The residual oil was dissolved in 5N HCl, washed with ether (x2) and the aqueous layer basified with aq. NaOH. Extraction with chloroform gave, on evaporation, an oil. Purification by column chromatography on silica using diethyl ether as eluent gave the analytically pure product (3.75 g) as an oil.

EXAMPLES 88-93

The following compounds were prepared by a method substantially as described in Example 87.

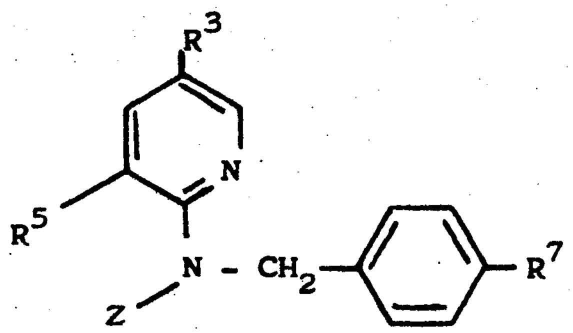

| Example No. | Z | $R^5$ | $R^7$ | R |
|---|---|---|---|---|
| 88 | $CH_3CH_2$ | H | Cl | H |
| 89 | $CH_3CH_2$ | H | $CH_3$ | $CH_3$ |
| 90 | $CH_3(CH_2)_2$ | H | $CH_3$ | $CH_3$ |
| 91 | $CH_3(CH_2)_3$ | H | $CH_3$ | $CH_3$ |
| 92 | $(CH_3)_2CHCH_2$ | H | $CH_3$ | $CH_3$ |
| 93 | H | Cl | H | $CH_3$ |

EXAMPLE 94

Preparation of 6-[N-methyl-N-(4-methylbenzyl)amino]-3-hydroxy methyl pyridine

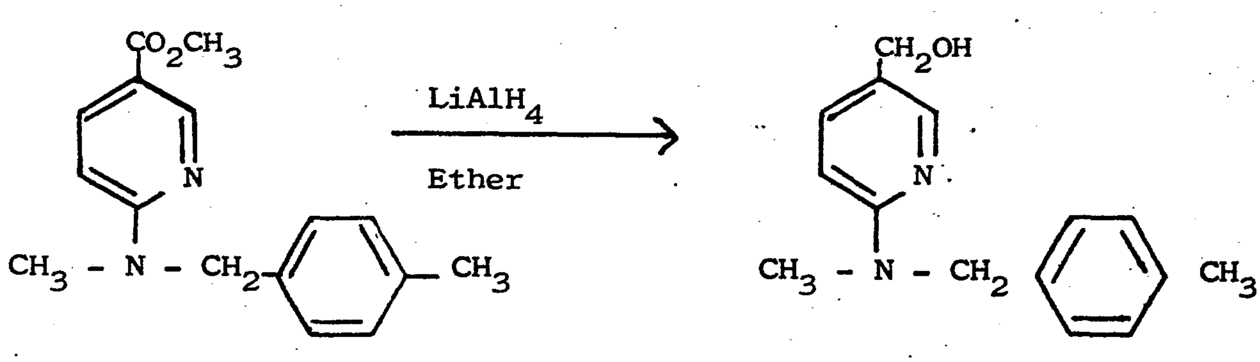


Excess lithium aluminium hydride (0.2 g) was placed in a 3-necked, 500 ml round-bottomed flask fitted with a mechanical stirrer, dropping funnel and condenser. The apparatus was thoroughly dry and flushed with nitrogen. Sodium-dried ether (50 ml) was added to the flask and the stirrer was started. The flask was cooled in ice and methyl-6-[N-methyl-N-(4-methyl benzyl)amino]nicotinate (2.5 g) dissolved in Na-dried ether (50 ml) was added dropwise. The reaction mixture was stirred at room temperature for one hour. Excess $LiAlH_4$ was decomposed by careful addition of ethyl acetate. The resulting solution was poured into dil. $H_2SO_4$. The organic layer was removed and the aqueous phase was basified with NaOH. The basic solution was extracted with ether. The ether extract was dried over $MgSO_4$ and concentrated to give a pale-yellow oil. The product was purified by column chromatography and distillation.

Boiling Point: 198° (0.6 mm Hg).

**BIOLOGICAL DATA:**

Hypoglycaemic assay in alloxan-diabetic mouse

For this assay, mice were made diabetic with alloxan five days before the experiments, drugs were dosed orally in 1% aq. carboxymethyl cellulose. Eleven animals were used for each treatment.

The table below shows the percentage lowering of compounds described in the present specification after 1 hour and 3 hours, compared with data for the known hypoglycaemic agent, Phenformin.

N.B.:

A standard system for indicating the significance of results with respect to control is used in the table.

The system is as follows:

| * | ** | *** | **** |
|---|---|---|---|
| $p<0.10$, | $p<0.05$, | $p<0.01$, | $p<0.001$ |

| Structure | Dose mM/kg | % Blood glucose lowering 1h | % Blood glucose lowering 3h | Phenformin 1 mM/kg po 1/3h |
|---|---|---|---|---|
| 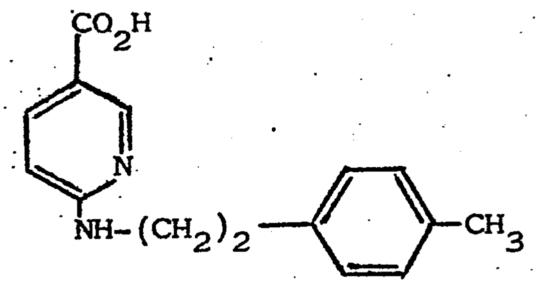 | 1.0<br>0.4 | 20.5**<br>4.2 | 37.2****<br>-11.8 | 21/28* |
| 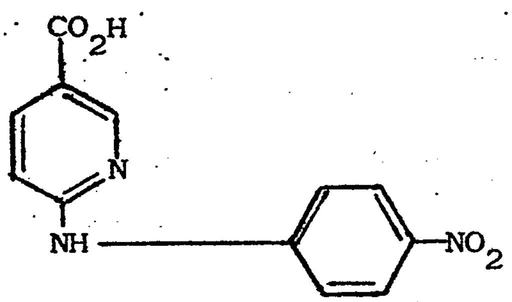 | 1.0<br>0.4 | 18.4***<br>8.4 | 27.5****<br>11.8 | 14**/11** |
| 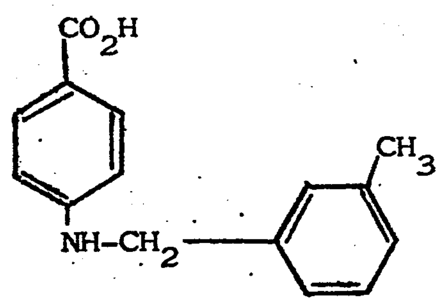 | 1.0<br>0.4 | 18.1*<br>13.3 | 26.3***<br>20.1**** | 11**/15 |
| 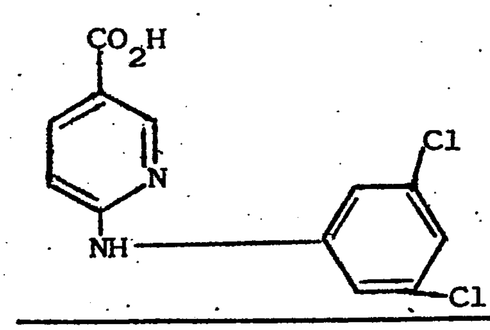 | 1.0<br>0.4 | 31.8***<br>29.8*** | 42.8****<br>28.5*** | 21/28* |
| 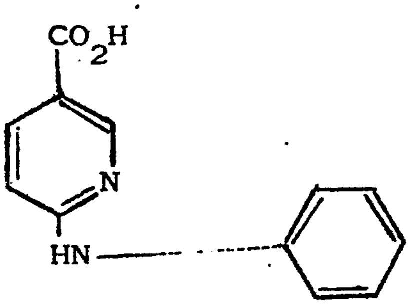 | 1.0<br>0.4 | 23.9<br>21.9*** | 22.6<br>20.4*** | 14/13** |

| Structure | Dose mM/kg | % Blood glucose lowering 1h | 3h | Phenformin 1 mM/kg po 1/3h |
|---|---|---|---|---|
| 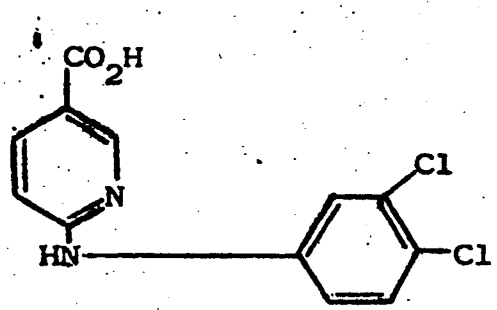 | 1.0<br>0.4 | 41.5****<br>30.0*** | 83.8****<br>45.6*** | 34****/26** |
| 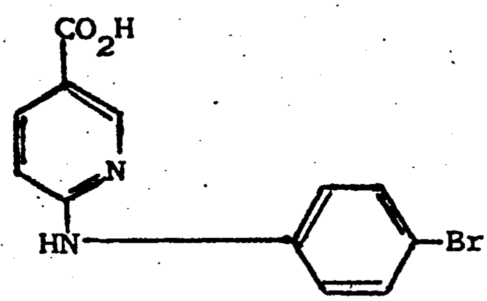 | 1.0<br>0.4 | 24.1****<br>27.8**** | 46.1****<br>45.5**** | 20****/16*** |
| 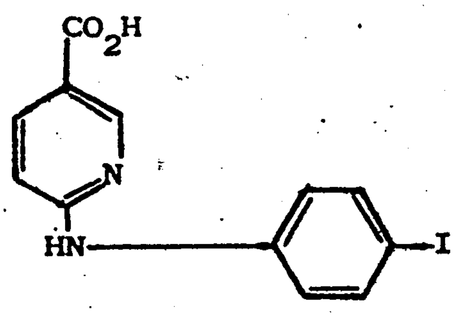 | 1.0<br>0.4 | 14.9***<br>4.9 | 42.4****<br>16.0** | 15***/13 |
| 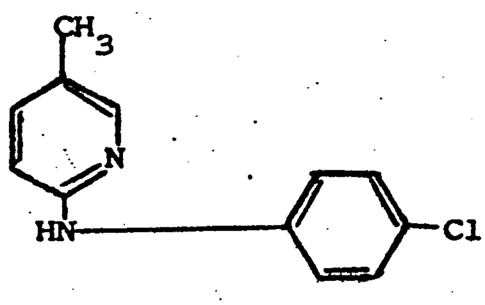 | 1.0<br>0.4 | 23.0**<br>13.5 | 20.3<br>2.7 | 26***/8 |
| 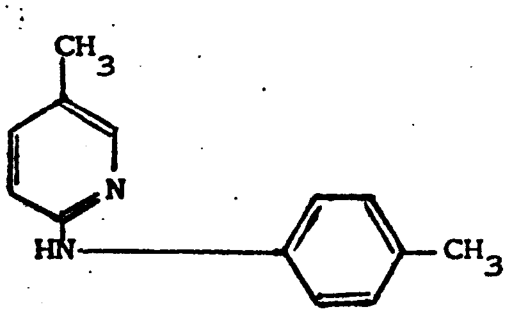 | 1.0<br>0.4 | 28.4***<br>13.9* | 7.5<br>-2.5 | 26***/8 |

| Structure | Dose mM/kg | % Blood glucose lowering 1h | 3h | Phenformin 1 mM/kg po 1/3h |
|---|---|---|---|---|
| 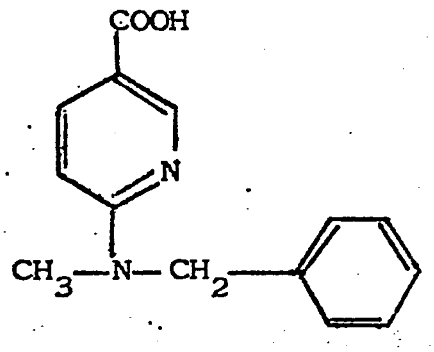 | 1.0<br>0.4 | 16.3**<br>5.0 | 14.5<br>17.8** | 14***/17** |
| 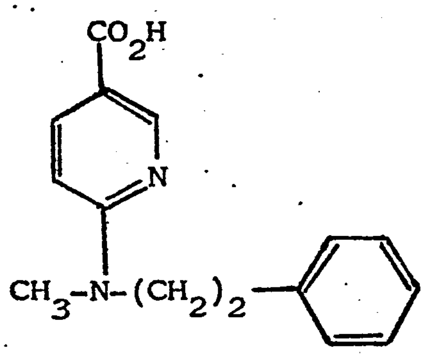 | 1.0<br>0.4<br>1.0<br>0.4 | 28.7****<br>16.7**<br>20.0**<br>12.0*** | 22.2**<br>16.9*<br>30.0****<br>15.0*** | 20***/23**<br><br>19****/21** |
| 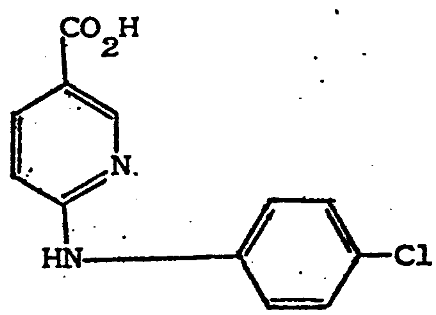 | 1.0<br>0.4 | 23.8****<br>35.6**** | 41.1****<br>54.7**** | 34****/26*** |
| 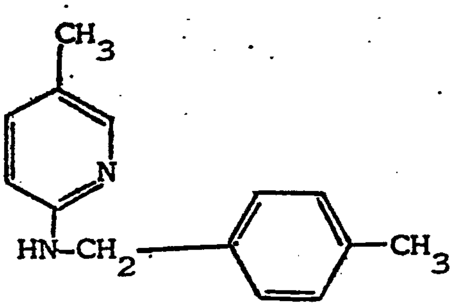 | 1.0<br>0.4 | 22.2**<br>-1.5 | 24.7****<br>12.4 | 22.5***/31.0** |

| Structure | Dose mM/kg | %Blood glucose lowering 1h | 3h | Phenformin 1 mM/kg; 1h/3h |
|---|---|---|---|---|
| 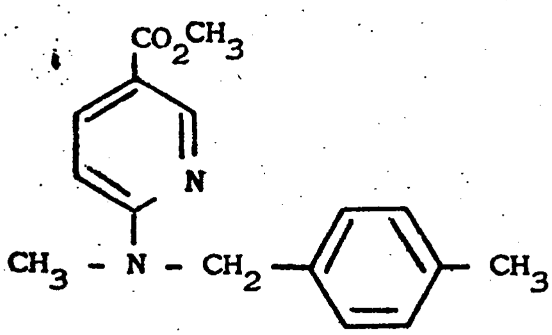 | 1.0<br>0.4 | 17*<br>5 | 20***<br>14* | 21****/27*** |
| 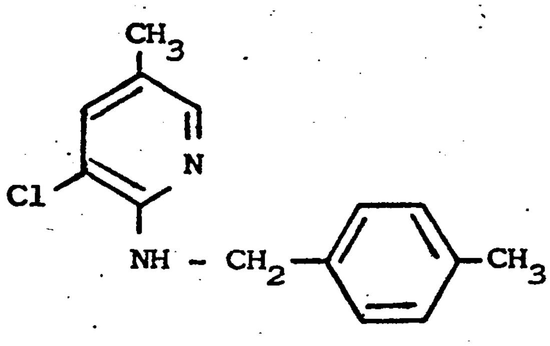 | 1.0<br>0.4 | 18***<br>16*** | 17<br>9 | 10/12 |
| 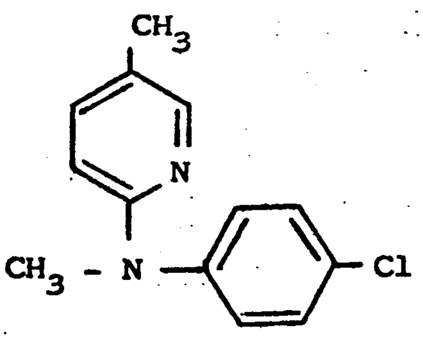 | 1.0<br>0.4 | 25**<br>8 | 46****<br>24*** | 9/26 |

| Compound | Dose mM/kg | % B. G. Lowering 1 h | 3 h | Phenformin 1mM/kg; 1h/3h |
|---|---|---|---|---|
| 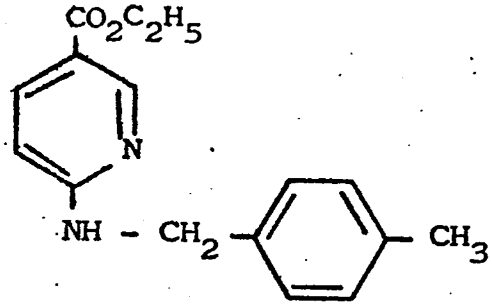 | 1.0<br>0.4 | $28^{***}$<br>4 | $20^{**}$<br>9 | 12/10 |
| 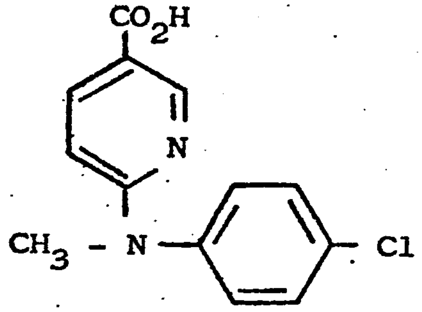 | 1.0<br>0.4 | $35^{****}$<br>$34^{****}$ | $52^{****}$<br>$43^{****}$ | $20^{***}/27^{****}$ |
| 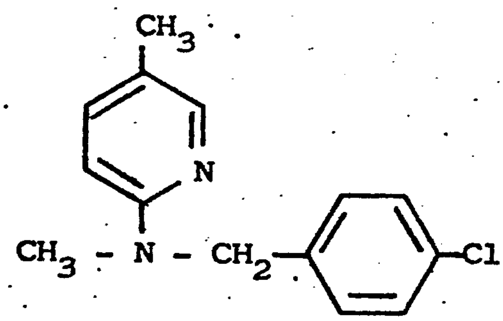 | 1.0<br>0.4 | $29^{****}$<br>$35^{****}$ | $39^{****}$<br>$39^{****}$ | 10/13 |
| 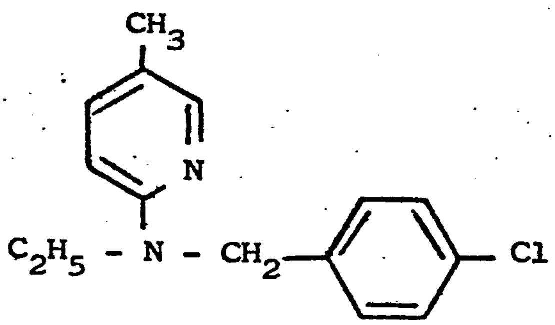 | 1.0<br>0.4 | $21^{***}$<br>19 | $27^{*}$<br>17 | 12/10 |
| 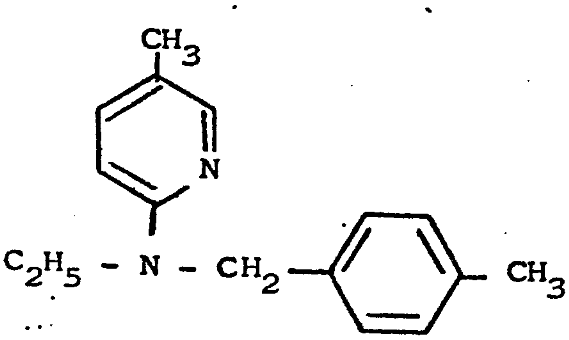 | 1.0<br>0.4 | $13^{**}$<br>7 | $25^{**}$<br>$15^{***}$ | $12/17^{**}$ |

| Compound | Dose mM/kg | % B. G. Lowering 1h | 3h | Phenformin 1mM/kg; 1h/3h |
|---|---|---|---|---|
| 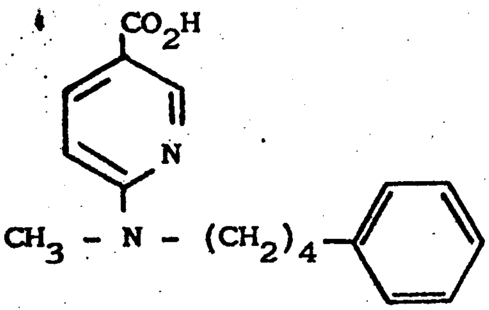 | 1.0 | 17** | 30** | 19*/36**** |
| | 0.4 | 19** | 14*** | |
| 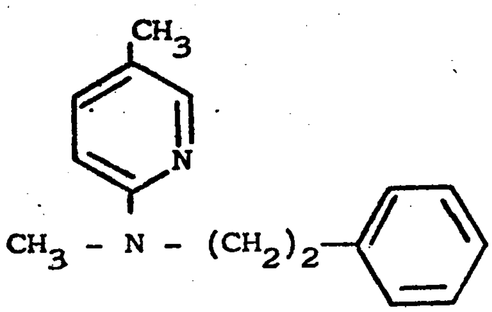 | 1.0 | 31*** | 28** | 19*/36**** |
| | 0.4 | 17 | 24*** | |
| 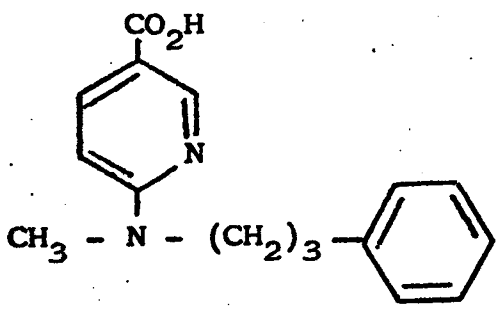 | 1.0 | 26**** | 32**** | 7/3 |
| | 0.4 | 15*** | 8 | |
| | 1.0 | 20**** | 30**** | 15*/17 |
| | 0.4 | 13** | 8 | |
| 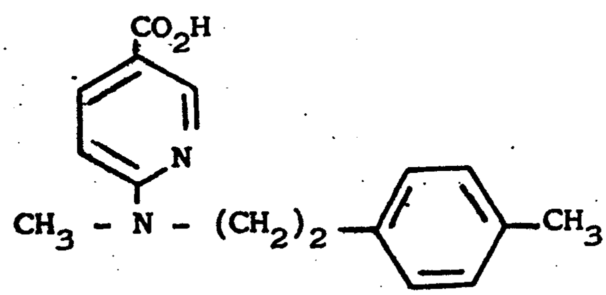 | 1.0 | 27*** | 17** | 12/10 |
| | 0.4 | 7 | 1 | |
| 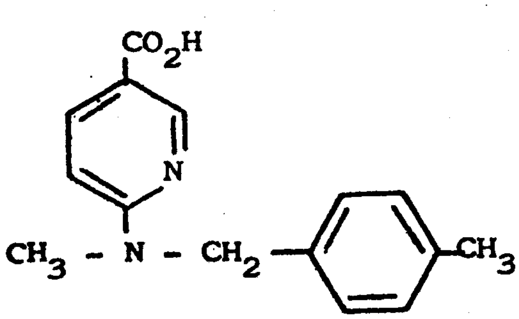 | 1.0 | 14** | 27**** | 10*/26** |
| | 0.4 | 13** | 27**** | |
| | 1.0 | 28**** | 37**** | 12/17** |
| | 0.4 | 20** | 35** | |

Hypolipidaemic assay

The hypocholesterolaemic and/or hypotriglyceridaemic effects of several compounds of the present invention were demonstrated in the following experiment;

Groups of 8 male albino rats (C.F.Y. strain), weighing approximately 150 g., were given a powdered commercially available diet (oxoid) to which compounds were added at level of of 0.1%. These diets were fed for seven days. The rats were then killed and their serum total cholesterol and triglyceride were measured by the Technicon Autoanalyser. The following table shows the results expressed in terms of percentage cholesterol lowering and percentage triglyceride lowering compared with controls.

Triglyceride and Cholesterol Lowering Results

| | % Cholesterol Lowering | % Triglyceride Lowering |
|---|---|---|
| 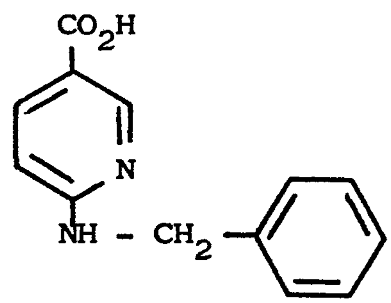 | 34 | 30 |
| 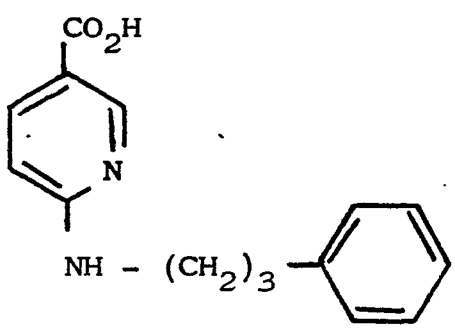 | 16 | 38 |

WHAT WE CLAIM IS: A

1. A pharmaceutical composition which comprises a pharmaceutically acceptable carrier together with, as active ingredient at least one compound of formula (I):

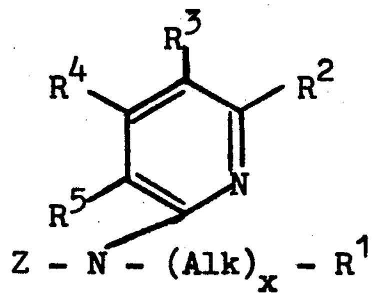

(I)

wherein $R^3$ is hydrogen or a carboxylic acid group or a pharmaceutically acceptable salt or ester of a carboxylic acid group; an alkyl group optionally substituted with one or more hydroxyl groups; or nitrile, formyl, tetrazolyl, or $C_{1-6}$ alkylcarbonyl group; and $R^2$ is hydrogen or $C_{1-6}$ alkyl and $R^4$ and $R^5$ are hydrogen, $C_{1-6}$ alkyl or halogen.

Z represents hydrogen, phenyl or $C_{1-6}$ alkyl optionally substituted with phenyl ;

Alk represents a straight or branched chain alkylene group having up to 12 carbon atoms;

x is zero or 1; and

$R^1$ represents phenyl or naphthyl, optionally substituted with up to three groups selected from $C_{1-6}$ alkyl, phenyl, halogen, $C_{1-6}$ alkoxy, amino, nitro, hydroxy, $C_{1-6}$ alkylamido, $C_{1-6}$ alkylcarbonyloxy, carboxy, $C_{1-6}$ alkoxycarbonyl, halo ( $C_{1-6}$ ) alkyl, oxo ( $C_{1-6}$ ) alkyl; or a pharmaceutically acid addition salt thereof.

2. A composition as claimed in claim 1 wherein the active ingredient is a compound of formula (II)

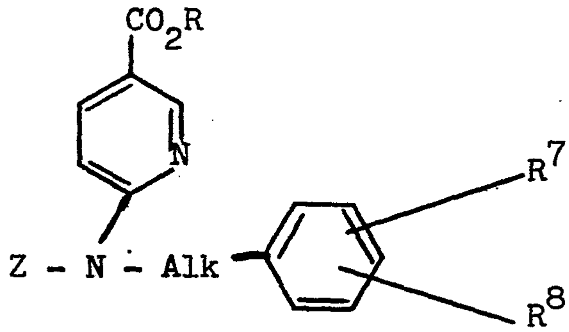

(II)

wherein Z and Alk are as defined in claim 1; R represents hydrogen, a pharmaceutically acceptable salting ion, a $C_{1-6}$ alkyl group or a readily hydrolysable ester; and $R_7$ and $R^8$ represent hydrogen, halogen, $C_{1-6}$ alkyl, $C_{1-6}$ alkoxy, or nitro.

3. A composition as claimed in 2 wherein $R^7$ is hydrogen and $R^8$ is hydrogen, halogen, $C_{1-6}$ alkyl or $C_{1-6}$ alkoxy.

4. A composition as claimed in claim 1 wherein $R^5$ is halogen.

5. A composition as claimed in claim 1 wherein the active ingredient is a compound of formula (III)

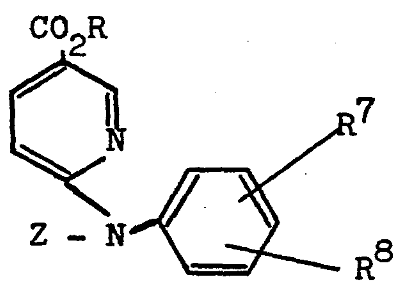

(III)

wherein Z is as defined in claim 1, R represents hydrogen, a pharmaceutically acceptable salting ion, a $C_{1-6}$ alkyl group or a readily hydrolysable ester; and $R^7$ and $R^8$ represent hydrogen, halogen, $C_{1-6}$ alkyl, $C_{1-6}$ alkoxy, nitro, carboxy or $C_{1-6}$ alkoxycarbonyl.

6. A composition as claimed in claim 5 wherein $R^7$ and $R^8$ represent hydrogen, halogen, $C_{1-6}$ alkyl or $C_{1-6}$ alkoxy.

7. A composition as claimed in claim 1 wherein the active ingredient is a compound of formula(IV):

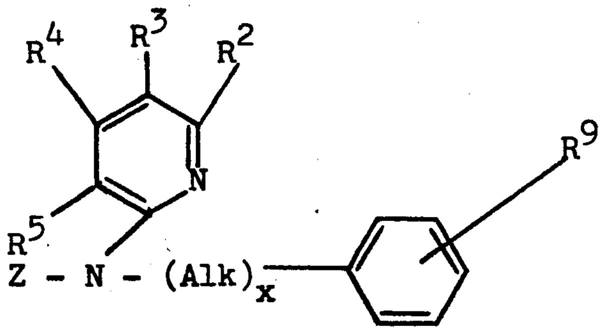

(IV)

wherein Z, Alk and x are as defined in claim 1, $R^2$, $R^3$, $R^4$ and $R^5$ are hydrogen, or $C_{1-6}$ alkyl and $R^9$ is hydrogen, halogen, $C_{1-6}$ alkyl or $C_{1-6}$ alkoxy.

8. A composition as defined in claim 7 wherein at least one of $R^2$, $R^3$, $R^4$, and $R^5$ is methyl.

9. A composition as claimed in any one of the claims 1 to 8 in the form of tablets, capsules, powders, granules, lozengers or sterile solutions or suspensions.

10. The use of a compound of formula (I) as defined in claim 1 for the treatment or control of diabetes.

11. A compound of formula (VII):

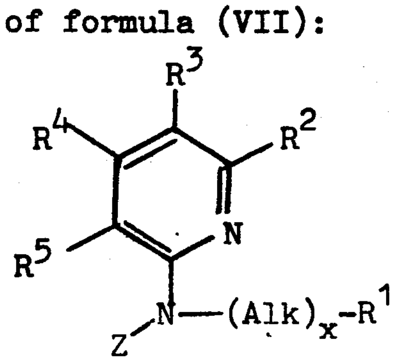

(VII)

wherein $R^3$ is hydrogen or a carboxylic acid group or a pharmaceutically acceptable salt or ester of a carboxylic acid group; an alkyl group optionally substituted with one or more hydroxyl groups; or nitrile, formyl, tetrazolyl, or $C_{1-6}$ alkylcarbonyl group; and $R^2$ is hydrogen or $C_{1-6}$ alkyl and $R^4$ and $R^5$ are hydrogen, $C_{1-6}$ alkyl or halogen.

Z represents hydrogen, phenyl or $C_{1-6}$ alkyl optionally substituted with phenyl;

Alk represents a straight or branched chain alkylene group having up to 12 carbon atoms;

x is zero or 1; and

$R^1$ represents phenyl or naphthyl, optionally substituted with up to three groups selected from $C_{1-6}$ alkyl, phenyl, halogen, $C_{1-6}$ alkoxy, amino, nitro, hydroxy, $C_{1-6}$ alkylamido, $C_{1-6}$ alkylcarbonyloxy, carboxy, $C_{1-6}$ alkoxycarbonyl, halo-( $C_{1-6}$ )-alkyl, oxo-( $C_{1-6}$ )-alkyl; or a pharmaceutically acceptable acid addition salt thereof; provided that :

(a) When x represent zero, then Z represents $C_{1-6}$ alkyl, phenyl or phenylalkyl;

(b) When x represents 1, Z is hydrogen, Alk is $-CH_2-$ and either $R^3$ or $R^4$ is methyl, then $R^1$ is other than phenyl.

12. A compound as claimed in claim 11 having the formula (II):

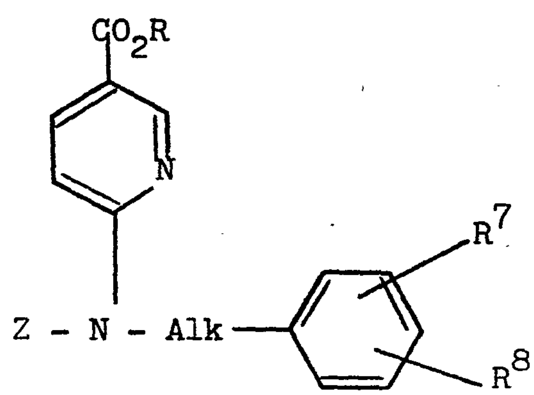

(II)

wherein Z and Alk are as defined in claim 11, R represents hydrogen, a pharmaceutically acceptable salting ion, a $C_{1-6}$ alkyl group or a readily hydrolysable ester; and $R^7$ and $R^8$ represent hydrogen, halogen, $C_{1-6}$alkyl, $C_{1-6}$alkoxy or nitro.

13. A compound as claimed in claim 12 wherein $R^7$ is hydrogen and $R^8$ is hydrogen, halogen, $C_{1-6}$ alkyl or $C_{1-6}$ alkoxy.

14. A compound as claimed in claim 11 having the formula (III):

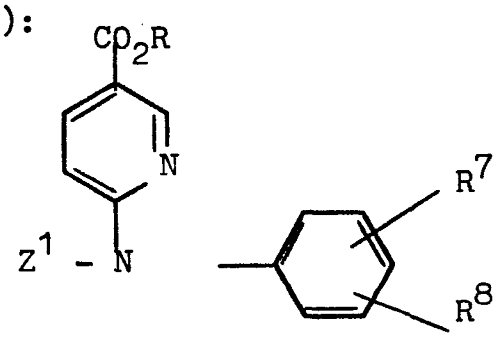

(III)

wherein $Z^1$ is phenyl or $C_{1-6}$ alkyl optionally substituted with phenyl, R represents hydrogen, a pharmaceutically acceptable salting ion, a $C_{1-6}$ alkyl group or a readily hydrolysable ester; and $R^7$ and $R^8$ represent hydrogen, halogen, $C_{1-6}$alkyl, $C_{1-6}$ alkoxy, nitro, carboxy or $C_{1-6}$ alkoxycarbonyl.

15. A compound as claimed in claim 14 wherein $R^7$ and $R^8$ represent hydrogen, halogen, $C_{1-6}$ alkyl or $C_{1-6}$ alkoxy.

16. A compound as claimed in claim 11 having the formula (IV):

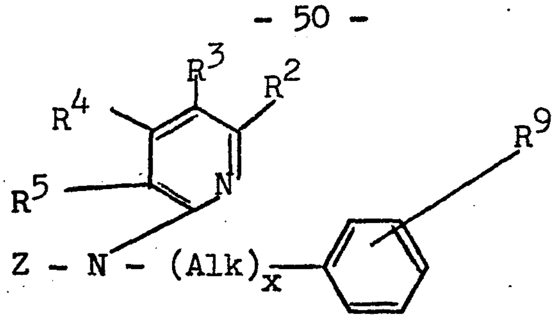

(IV)

wherein $R^2$, $R^3$, $R^4$ and $R^5$ are hydrogen, or $C_{1-6}$ alkyl;

$R^9$ is hydrogen, halogen, $C_{1-6}$ alkyl or $C_{1-6}$ alkoxy;

Alk represents a $C_{2-6}$ straight or branched alkylene chain; and

either x is zero and Z is $C_{1-6}$ alkyl, phenyl or benzyl;

or x is 1 and Z is hydrogen, $C_{1-6}$ alkyl, phenyl or benzyl.

17. A compound as claimed in claim 16 wherein one of $R^2$, $R^3$, $R^4$ and $R^5$ is methyl.

18. A process for the preparation of a compound as claimed in claim 11 which process comprises:

a) reacting an amine of formula (VIII) with a halide of formula (IX):

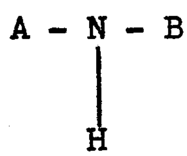

Hal - D

(VIII) (IX)

wherein " Hal " represents halogen; one group A, B or D represents a group of formula (X):

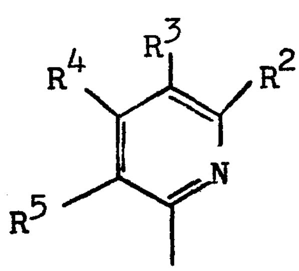

(X)

[ wherein $R^2$, $R^3$,$R^4$ and $R^5$ are as defined in claim 11 ] one group A, B or D represents a group of formula (XI);

$$-(\text{Alk})_x - R^1 \qquad \text{(XI)}$$

[ wherein Alk, x and $R^1$ are as defined in claim 11 ]; and the third group A, B, or D represents the group Z as defined in claim 11;

b) reacting an amine of formula (XII) with a compound of formula (XIII):

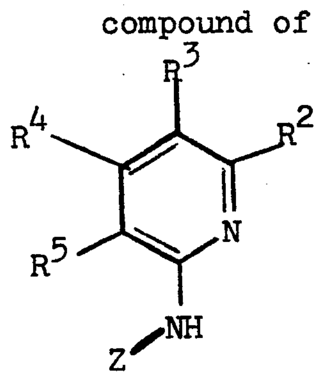

$HO-(Alk)_x-R^1$

(XII) (XIII)

wherein $R^1$, $R^2$, $R^3$, $R^4$, $R^5$, Z, Alk and x are as defined in claim 11;

c) when x is 1 and Alk represents methylene reducing a compound of formula (XIV):

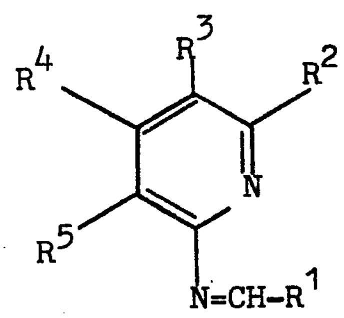

(XIV)

wherein $R^1$, $R^2$, $R^3$, $R^4$ and $R^5$ are as defined in claim 11;

d) for compounds of formula (VII) with a $CH_2$ group adjacent the amino nitrogen, reducing a ketone of formula (XVII A) or (XVII B):

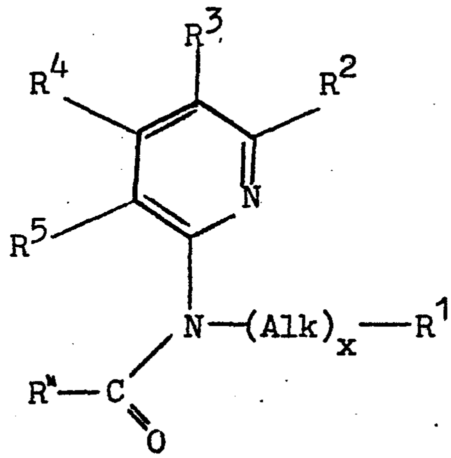

(XVII A)

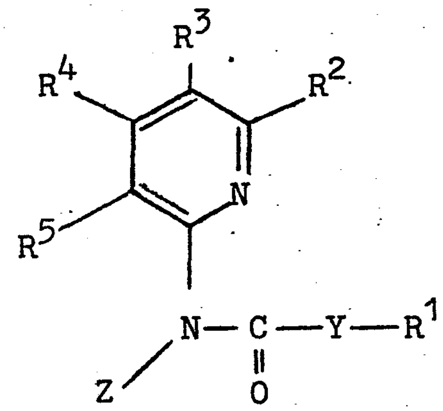

(XVII B)

wherein $R^{11}$ represents a $C_{1-5}$ alkyl group optionally substituted with phenyl, Y represents a bond or a $C_{1-11}$ alkylene group, and $R^1$, $R^2$, $R^3$, $R^4$, $R^5$, Alk, x and Z are as defined in claim 11;

and after step a), b), c) or d), optionally converting one group $R^3$ or $R^1$ to a different such group.

19. A compound selected from:

6- [ N-methyl-N-( 4-methylbenzyl ) amino ] -3-picoline
6- [ N-methyl-N-( 4-chlorophenyl ) amino ] nicotinic acid
6- [ N-methyl-N-( 4 chlorophenyl ) amino ] -3-picoline
6- [ N-methyl-N-( 3,4 dimethylphenyl ) amino ] -3-picoline
6- [ N-methyl-N-( 4-methylbenzyl ) amino ] nicotinic acid and it's methyl ester
6- [ N-methyl-N-( 4-chlorobenzyl ) amino ] -3-picoline
6- [ N-methyl-N-( 4-carboxybenzyl ) amino ] -3-picoline

European Patent Office

# PARTIAL EUROPEAN SEARCH REPORT

which under Rule 45 of the European Patent Convention shall be considered, for the purposes of subsequent proceedings, as the European search report

Application number EP 78 30 0190

## DOCUMENTS CONSIDERED TO BE RELEVANT

| Category | Citation of document with indication, where appropriate, of relevant passages | Relevant to claim |
|---|---|---|
| X | NL - A - 67 07290 (EGYE SULT GYOGYSZER)<br>* Examples 10,11; claims; page 3, lines 16-20 *<br>& GB - 1 188 154<br>-- | 1,7,9, 11,18, 19 |
| X | GB - A - 1 420 987 (CASSELLA)<br>* Page 10, lines 61-64; claims; examples 216,220 *<br>-- | 1,7-9, 11,18, 19 |
| X | GB - A - 1 191 302 (DEUTSCHE GOLD UND SILBER)<br>* Page 1, lines 39-41; claims *<br>-- | 1-4, 7-9, 11-13, 16-18 |
| X | NL - A - 65 11104 (DEUTSCHE GOLD UND SILBER)<br>* Page 2, lines 8-10; example 14; claims *<br>--<br>./. | 1,4-9, 18-19 |

**CLASSIFICATION OF THE APPLICATION (Int. Cl.²)**

C 07 D 213/74
C 07 D 213/80
C 07 D 213/85
C 07 D 401/04
A 61 K 31/44
A 61 K 31/455
//(C 07 D 401/04
C 07 D 257/04
C 07 D 213/74)

**TECHNICAL FIELDS SEARCHED (Int.Cl.²)**

C 07 D 213/74
C 07 D 213/80
C 07 D 213/85
C 07 D 401/04
C 07 D 213/79
A 61 K 31/44
A 61 K 31/455

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:

Claims searched incompletely:

Claims not searched: 10

Reason for the limitation of the search: Method for treatment of the human or animal body by surgery or therapy (See article 52(4) of the European Patent Convention)

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| Place of search | Date of completion of the search | Examiner |
|---|---|---|
| The Hague | 01-11-1978 | NUYTS |

EPO Form 1505.1 06.78

European Patent Office

# PARTIAL EUROPEAN SEARCH REPORT

Application number
EP 78 30 0190
-2-

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| X | DE - A - 2 141 418 (ROEMMERS)<br>* Page 2, paragraph 1; claims * | 1,4,7-9,18 | |
| X | DE - B - 1 119 274 (DR. RASCHIG)<br>* Page 1, lines 19-26; examples 11-14; claims * | 1,7,9,11,18 | |
| X | US - A - 3 450 707 (D.M. BAILEY)<br>* Column 1, lines 50-55; examples; claims * | 1,7-9,16-18 | TECHNICAL FIELDS SEARCHED (Int. Cl.²) |
| X | CHEMICAL ABSTRACTS, vol. 86 (1977) p 100, 84340x<br>L.G. GRIFFIS et al."The acute toxicity of 2 benzylaminopyridine"<br>& TOXICOL.APPL.PHARMACOL. 1976, 38(3) 639-41 | 1,7,9,11 | |
| X | DIE PHARMAZIE, 32 (3) 1977, Berlin, P 149-50<br>A.H. ABO-SIER et al."Synthesis of some 3,4,5-trimethoxybenzylderivatives of certain amino compounds likely to possess CNS activity"<br>* Compound 1a; page 149, paragraph 1; experiment 3.1; pages 149-150 * | 1,7,9,11,18 | |
| X | CHEMICAL ABSTRACTS, vol. 83, 1975, 96954b p 553<br>J. DELARGE "Synthesis of nonsteroidal antiinflammatory substances"<br>& MEM.ACAD.R.MED.BELG. 1974, 47(3), 131-120<br>* Abstract; Chemical Abstracts 9th ./. | 1,5,6,9,18 | |

European Patent Office

**PARTIAL EUROPEAN SEARCH REPORT**

Application number EP 78 30 0190



| Category | DOCUMENTS CONSIDERED TO BE RELEVANT<br>Citation of document with indication, where appropriate, of relevant passages | Relevant to claim | CLASSIFICATION OF THE APPLICATION (Int. Cl.²) |
| --- | --- | --- | --- |
| | Collect. Chemical Substances Index 9CS21, p 33816 CS<br>-- | | |
| X | BULLETIN DE LA SOCIETE CHIMIQUE DE FRANCE, 1975, (3-4) p 576-82<br>M. DOME et al." No. 108 Alkylations en milieu aqueux par les halogénures aminés ou amidés. II Réactions avec quelques nucléophiles"<br>* Paragraph 1; page 576; page 578, compounds 18b,d; page 581, left-hand column; page 580 "II Actor. cytotoxique" *<br>-- | 1,7,9,11,18 | TECHNICAL FIELDS SEARCHED (Int. Cl.²) |
| | IL FARMACO, EDIZIONE SCIENTIFICA, 31 (1976) (2) p. 105-11<br>G. BETTONI et al. "Determinazione della confrigurazione assoluta di composti amminici aventi interesse farmaceutico attraverso misure di dicroismo circolare"<br>* Page 105, paragraph 1; page 106, formulae 1a, A,B; page 108, compounds IIIa,IVa,VIa,VIIa, VIIIa; pages 110-111; experiment-part *<br>-- | 1,7,9,11,16,18 | |
| X | CHEMICAL ABSTRACTS, vol. 53 (1959) 18951c<br>H. FUERST et al."Alkylamino pyridines and alkyl pyridine ethers"<br>* Abstract *<br>-- | 11,16-18 | |
| X | GB - A - 1 270 363 (RHONE POULENC)<br>* Claims *<br>-- | 11,16-19 | |
| X | GB - A - 1 405 308 (BASF)<br>* Claims; page 8, table 2, number 29; page 9, table 2, number 43 *<br>-- ./. | 11,18 | |

European Patent Office

# PARTIAL EUROPEAN SEARCH REPORT

Application number
EP 78 30 0190
-4-

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| X | FR - A - 2 340 934 (BASF)<br>* Claims; page 10, examples 48-50 * | 11,16,<br>18 | |
| X | US - A - 2 784 138 (R. WEGLER)<br>* Claims; examples 7-8; column 1, line 64 - column 2, line 30 * | 11,18 | |
| X | CHEMICAL ABSTRACTS, vol. 87 (1977) p. 544, 117193c<br>G. BETTONI et al."The chiroptical properties of some substituted N-(pyridyl) derivatives of amines"<br>& GAZZ.CHIM.ITAL. 1977, 107(1-2) 111-16<br>* Abstract; chemical abstracts chemical substance index vol. 87 (1977) p. 4843 CS * | 11,16,<br>18 | TECHNICAL FIELDS SEARCHED (Int. Cl.²) |
| X | CHEMISCHE BERICHTE, 109 (1976) p. 3661-7<br>W. STADLBAUER et al." Synthese mesoionischer S-Triazine"<br>* Page 3662, formula 1c; experiment; page 3664, paragraph 1 * | 11,18 | |
| X | BULLETIN OF THE CHEMICAL SOCIETY OF JAPAN, vol. 49 (10), 1976, p. 2770-74<br>Y. TAKAHASHI et al."Studies on the tantomerism of 2-anilinopyridine and related heterocycles"<br>* Page 2770, experimental paragraph 1; page 2771, table 1, compound 3 * | 11,18 | |
| | ./. | | |

European Patent Office

**PARTIAL EUROPEAN SEARCH REPORT**

Application number
EP 78 30 0190
-5-

| DOCUMENTS CONSIDERED TO BE RELEVANT | | | CLASSIFICATION OF THE APPLICATION (Int. Cl.) |
|---|---|---|---|
| Category | Citation of document with indication, where appropriate, of relevant passages | Relevant to claim | |
| X | TETRAHEDRON, Pergamon Press (1973) 29(2) p. 419-24 S.V. KESSAR et al."New routes to condensed polynuclear compounds IX" <br> * Page 420, compound 11; page 423, paragraph 4 * <br> -- | 11,18 | |
| X | FR - A - 2 288 798 (POPESCU) <br> * Page 2, table 2, compounds 6-9 * <br> -- | 11 | |
| | | | TECHNICAL FIELDS SEARCHED (Int. Cl.) |
| X | US - A - 3 535 328 (J. ZIELINSKI) <br> * Column 19, compound 20; column 22, line 4 * <br> -- | 11,18 | |
| X | FR - A - 2 155 922 (ARIES) <br> * Page 2, line 34 - page 4, line 13; page 4, lines 28,30 * <br> -- | 11,16 | |
| | CHEMICAL ABSTRACTS, vol. 82 (1975) p. 446, 16223d TORTORELLA V. et al. "Influence of additional aromatic chromophores on the chiroptical properties of N-(2 pyridyl N-oxide) amino derivatives" <br> & GAZZ.CHIM.ITAL. 1973, 103 (10-12) 1083-98 <br> * Abstract; chemical abstracts, 9th collective index, chemical substances 9CS20, p. 33543 CS - 33549 CS * <br> -- | 11,16, 18 | |

./.

European Patent Office

# PARTIAL EUROPEAN SEARCH REPORT

Application number
EP 78 30 0190
-6-

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| | TETRAHEDRON, Pergamon Press 1973, vol. 29, p. 1345-52<br>V. TORTORELLA et al."NMR-Investigation on the conformational properties of N-(2-pyridyl-N-oxide)-amino derivatives<br>* Page 1346 compounds 2-4, 2a-4a; page 1351, experiment *<br>---- | 11,16 | |
| | | | TECHNICAL FIELDS SEARCHED (Int. Cl.²) |